# EUROPEAN PATENT APPLICATION

(11) **EP 4 491 619 A1**
(43) Date of publication of application: **15.01.2025**
(21) Application number: 23766651.6
(22) Date of filing: 01.03.2023
(51) Int. Cl.: C07D 279/18, G01N 27/327

(54) **ELECTRON ACCEPTOR, AND REAGENT LAYER AND SENSOR EACH USING SAME**

(30) Priority: 07.03.2022 JP 2022034824
(71) Applicant: PHC Holdings Corporation, Tokyo 100-8403 (JP)
(72) Inventor: HANEDA, Keigo, Ehime 7910395 (JP); TAKAHARA, Yoshifumi, Ehime 7910395 (JP)
(74) Representative: Eisenführ Speiser
(86) International application number: PCT/JP2023/007472
(87) International publication number: WO 2023/171473

(57) **Abstract**

The present invention provides a redox polymer for forming an electrochemical sensor that is suitable for use in measurement over a long period (continuous monitoring) and excellent in stability, or a redox mediator constituting the same. The redox mediator provided by the present invention is a phenothiazine-based compound having a hydrophilic moiety introduced therein, the compound being represented by the general formula (2A) or (2B). The redox polymer provided by the present invention is a phenothiazine-based compound bound to a polymer, the compound being represented by the general formula (3A) or (3B). In the formulas, R⁹ and R¹⁰ are both substituents, or one of these moieties is a substituent, and the other moiety is a hydrogen atom, R(L)¹, R(L)², and R(L)⁴ to R(L)⁸ are each independently a predetermined group or atom, (QP)¹, (QP)², and (QP)⁴ to (QP)^{B} are each independently a polymeric structure derived from a high molecular weight polymer which is present when their corresponding R(L)¹, R(L)², and R(L)⁴ to R(L)⁸ are a specific group.

## Description

### Technical Field

The present disclosure relates to an electron acceptor, and a reagent layer and a sensor each using the same. More specifically, the present invention relates to an electron acceptor (redox mediator) which is a compound having a phenothiazine structure, and a reagent layer comprising the electron acceptor, and a sensor having the reagent layer.

### Background Art

Heretofore, sensors that allow proteins to act on analytes in samples and measure the analytes have been known. Examples of such a sensor include electrochemical sensors using an enzyme, for example, glucose sensors prepared using glucose oxidoreductase and optionally a redox mediator (oxidation-reduction material that mediates electron transfer) or a redox polymer (polymer having the redox mediator bound thereto via a linker or the like). The glucose sensors are used for, for example, self examination of blood glucose levels. Although conventional sensors generally involve collecting a very small amount of blood and using this blood as a sample, electrochemical glucose sensors of embedded type which are embedded in a living body to continuously measure glucose in blood or in the interstitium have also been developed in recent years. The glucose sensors are also used for measuring glucose in samples outside biological materials, for example, in media. Such glucose sensors generally perform the measurement of glucose concentrations in samples continuously or semi-continuously for a long time such as several days to several weeks.

Continuous monitoring sensors typified by such electrochemical glucose sensors require measurement for a long time, and sensor materials (enzyme, mediator, etc.) used are therefore desired to have high stability. As for a technique of improving the stability of an enzyme itself in response to such a demand, for example, Patent Literature 1 describes a FAD-dependent glucose dehydrogenase variant that is superior in preservation stability (long-term stability) to glucose oxidase conventionally used, i.e., has high residual enzymatic activity after a lapse of a long time in the body or the like, and has a specific amino acid sequence, and a continuous glucose monitoring device using the same. As for a technique related to improvement in the stability of a redox mediator and a redox polymer, for example, Patent Literature 2 illustrates a redox mediator consisting of a transition metal complex having high stability during use and during preservation, and a polymer having the redox mediator bound thereto.

### Citation List

### Patent Literature

Patent Literature 1: Japanese Patent Laid-Open No. 2018-201486
Patent Literature 2: U.S. Patent No. 8,444,834

### Summary of Invention

### Technical Problem

In one aspect, an object of the present invention is to provide an electrochemical sensor that is suitable for use in measurement over a long period (continuous monitoring) and excellent in stability. In another aspect, an object of the present invention is to provide a redox polymer for forming a reagent layer of the electrochemical sensor as described above or a redox mediator constituting the same.

### Solution to Problem

The present inventors have found that a phenothiazine-based compound having an iminium cation at the 3-position of a phenothiazine skeleton is excellent in functional stability of electron transfer even when a hydrophilic moiety (e.g., a structure containing a polyethylene glycol chain) is bound to a position different therefrom (e.g., the 6-position) in the skeleton or when a high molecular weight polymer is bound to the compound, and is suitable as a redox mediator for preparing an electrochemical sensor for use in measurement over a long period in a wet environment such as a living body or a medium.

Specifically, the present invention encompasses at least the following items.
[1] A phenothiazine-based compound having a hydrophilic moiety introduced therein, the compound being represented by the general formula (2A) or (2B): 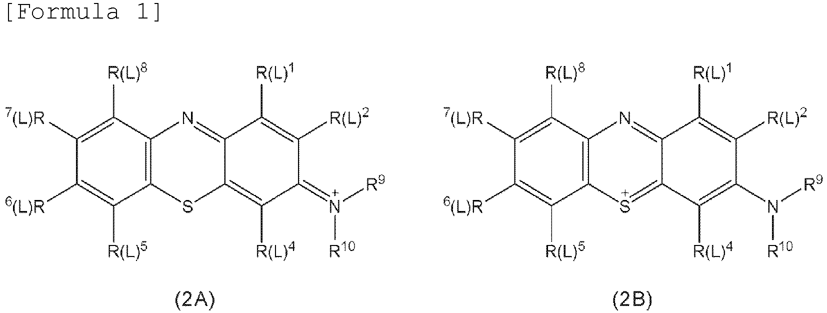 wherein
   R⁹ and R¹⁰ are both substituents, or one of these moieties is a substituent, and the other moiety is a hydrogen atom,
   R(L)¹, R(L)², and R(L)⁴ to R(L)⁸ are each independently [A] a hydroxy group optionally having a substituent, a carboxy group optionally having a substituent, an amino group optionally having a substituent, a linear or branched saturated or unsaturated hydrocarbon group having 1 to 6 carbon atoms and optionally having a substituent, an acyl group optionally having a substituent, or a phenyl group optionally having a substituent, [B] a group formed by the binding of a compound for hydrophilic moiety introduction to the binding substituent selected from [A], or [C] a hydrogen atom or a halogen atom,
   on the proviso that at least one of R(L)¹, R(L)², and R(L)⁴ to R(L)⁸ is the group [B].
[2] The phenothiazine-based compound having a hydrophilic moiety introduced therein according to item 1, wherein the substituent represented by each of R⁹ and R¹⁰ is an alkyl group having 1 to 6 carbon atoms.
[3] The phenothiazine-based compound having a hydrophilic moiety introduced therein according to item 1 or 2, wherein at least one of R(L)¹, R(L)², and R(L)⁴ to R(L)⁸ is a group formed by the binding of a compound for hydrophilic moiety introduction to a binding substituent having at least one reactive group selected from the group consisting of a carboxy group or an active ester form thereof, an amino group, a thiol group, a formyl group, an epoxy group, and a maleimide group.
[4] The phenothiazine-based compound having a hydrophilic moiety introduced therein according to any one of items 1 to 3, wherein at least one of R(L)¹, R(L)², and R(L)⁴ to R(L)⁸ is a group formed by the binding of a compound for hydrophilic moiety introduction to a binding substituent which is an amino group having a substituent, the binding substituent being represented by -NR¹¹R¹² (wherein one of R¹¹ and R¹² is a reactive N substituent, and the other moiety is a hydrogen atom or a nonreactive N substituent).
[5] The phenothiazine-based compound having a hydrophilic moiety introduced therein according to any one of items 1 to 4, wherein at least R(L)⁶ is a group formed by the binding of a compound for hydrophilic moiety introduction to the binding substituent.
[6] The phenothiazine-based compound having a hydrophilic moiety introduced therein according to any one of items 1 to 5, wherein the compound is represented by the general formula (2A-2) or (2B-2): 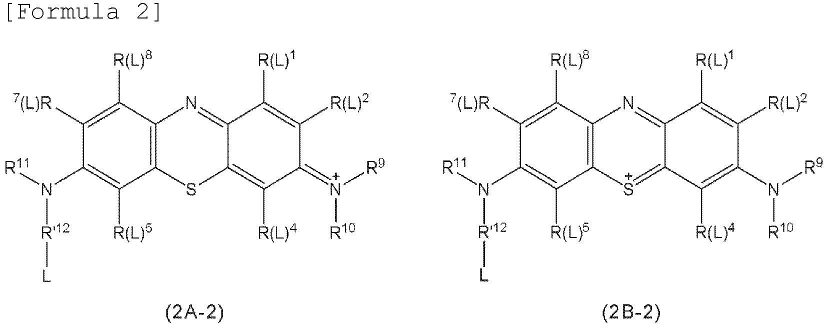 wherein R¹¹ is a hydrogen atom or a nonreactive N substituent, R'¹² is a group derived from a reactive N substituent, L is the hydrophilic moiety derived from the compound for hydrophilic moiety introduction, and the other symbols are as defined in the general formulas (2A) and (2B).
[7] The phenothiazine-based compound having a hydrophilic moiety introduced therein according to any one of items 1 to 6, wherein the hydrophilic moiety derived from the compound for hydrophilic moiety introduction comprises at least one member selected from the group consisting of a hydroxy group, an amino group, a carboxy group, a sulfo group, a quaternary ammonium cation, and groups derived therefrom.
[8] The phenothiazine-based compound having a hydrophilic moiety introduced therein according to any one of items 1 to 7, wherein the hydrophilic moiety derived from the compound for hydrophilic moiety introduction comprises an oxyethylene chain.
[9] The phenothiazine-based compound having a hydrophilic moiety introduced therein according to any one of items 1 to 8, wherein the hydrophilic moiety derived from the compound for hydrophilic moiety introduction has at least one reactive group selected from the group consisting of a carboxy group or an active ester form thereof, an amino group, a thiol group, a formyl group, an epoxy group, and a maleimide group.
[10] A phenothiazine-based compound bound to a polymer, the compound being represented by the general formula (3A) or (3B): 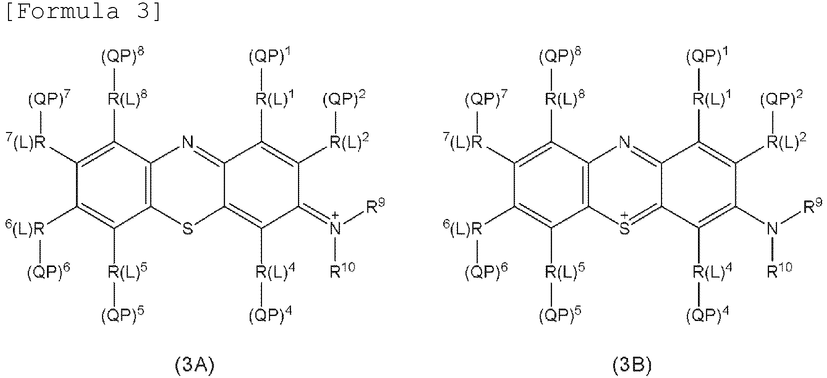 wherein
   R⁹ and R¹⁰ are both substituents, or one of these moieties is a substituent, and the other moiety is a hydrogen atom,
   R(L)¹, R(L)², and R(L)⁴ to R(L)⁸ are each independently [A] a hydroxy group optionally having a substituent, a carboxy group optionally having a substituent, an amino group optionally having a substituent, a linear or branched saturated or unsaturated hydrocarbon group having 1 to 6 carbon atoms and optionally having a substituent, an acyl group optionally having a substituent, or a phenyl group optionally having a substituent, [B] a group formed by the binding of a compound for hydrophilic moiety introduction to the binding substituent selected from [A], [C] a hydrogen atom or a halogen atom, or [D] a group derived from either the binding substituent selected from [A] or the group [B],
   (QP)¹, (QP)², and (QP) ⁴ to (QP) ⁸ are each independently a polymeric structure derived from a high molecular weight polymer which is present when their corresponding R(L)¹, R(L)², and R(L)⁴ to R(L)⁸ are the group [D], and
   on the proviso that at least one of (QP)¹, (QP)², and (QP)⁴ to (QP)⁸ is present.
[11] The phenothiazine-based compound bound to a polymer according to item 10, wherein the substituent represented by each of R⁹ and R¹⁰ is an alkyl group having 1 to 6 carbon atoms.
[12] The phenothiazine-based compound bound to a polymer according to item 10 or 11, wherein at least one of R(L)¹, R(L)², and R(L)⁴ to R(L)⁸ is a group derived from either a binding substituent having at least one reactive group selected from the group consisting of a carboxy group or an active ester form thereof, an amino group, a thiol group, a formyl group, an epoxy group, and a maleimide group, or a group formed by the binding of a compound for hydrophilic moiety introduction to the binding substituent.
[13] The phenothiazine-based compound bound to a polymer according to any one of items 10 to 12, wherein at least one of R(L)¹, R(L)², and R(L)⁴ to R(L)⁸ is a group derived from either a binding substituent which is an amino group having a substituent, the binding substituent being represented by -NR¹¹R¹² (wherein one of R¹¹ and R¹² is a reactive N substituent, and the other moiety is a hydrogen atom or a nonreactive N substituent), or a group formed by the binding of a compound for hydrophilic moiety introduction to the binding substituent.
[14] The phenothiazine-based compound bound to a polymer according to any one of items 10 to 13, wherein at least R(L)⁶ is a group derived from either a binding substituent or a group formed by the binding of a compound for hydrophilic moiety introduction to the binding substituent.
[15] The phenothiazine-based compound bound to a polymer according to any one of items 10 to 14, wherein at least R(L)⁶ is a group derived from a group formed by the binding of a compound for hydrophilic moiety introduction to the binding substituent.
[16] The phenothiazine-based compound bound to a polymer according to any one of items 10 to 15, wherein the compound is represented by the general formula (3A-2) or (3B-2) : 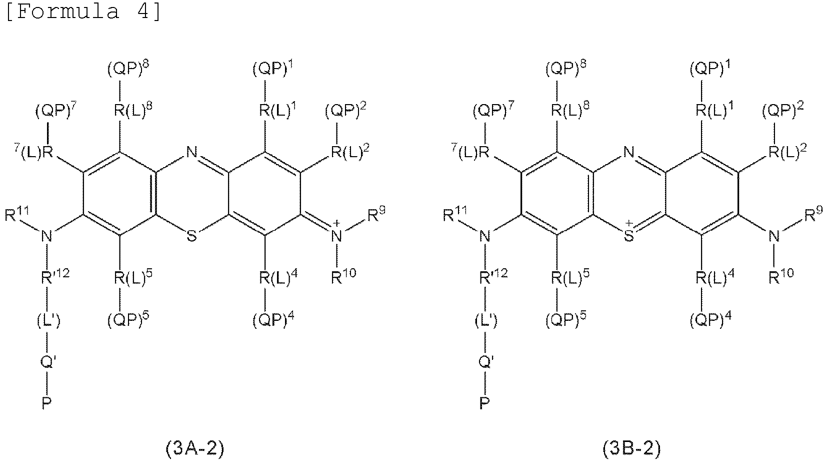 wherein R¹¹ is a hydrogen atom or a nonreactive N substituent, R'¹² is a group derived from a reactive N substituent, (L') is optionally present and is a group derived from a compound for hydrophilic moiety introduction, Q' is a group derived from a reactive group of a high molecular weight polymer, P is a backbone of the high molecular weight polymer, and the other symbols are as defined in the general formulas (3A) and (3B).
[17] The phenothiazine-based compound bound to a polymer according to any one of items 10 to 16, wherein the hydrophilic moiety derived from the compound for hydrophilic moiety introduction comprises at least one member selected from the group consisting of a hydroxy group, an amino group, a carboxy group, a sulfo group, a quaternary ammonium cation, and groups derived therefrom.
[18] The phenothiazine-based compound bound to a polymer according to any one of items 10 to 17, wherein the hydrophilic moiety derived from the compound for hydrophilic moiety introduction comprises an oxyethylene chain.
[19] The phenothiazine-based compound bound to a polymer according to any one of items 10 to 18, wherein at least one of R(L)¹, R(L)², and R(L)⁴ to R(L)⁸ is a group derived from a group formed by the binding of a compound for hydrophilic moiety introduction having at least one reactive group selected from the group consisting of a carboxy group or an active ester form thereof, an amino group, a thiol group, a formyl group, an epoxy group, and a maleimide group to the binding substituent.
[20] The phenothiazine-based compound bound to a polymer according to any one of items 10 to 19, wherein the high molecular weight polymer is at least one member selected from the group consisting of an amino acid-based polymer, an imine-based polymer, an ethylene-based polymer, and a protein.
[21] The phenothiazine-based compound bound to a polymer according to any one of items 10 to 20, wherein the high molecular weight polymer is a cationic high molecular weight polymer.
[22] A reagent layer comprising a phenothiazine-based compound having a hydrophilic moiety introduced therein according to any one of items 1 to 9, or a phenothiazine-based compound bound to a polymer according to any one of items 10 to 21.
[23] The reagent layer according to item 22, further comprising an oxidoreductase that oxidizes or dehydrogenates an analyte.
[24] The reagent layer according to item 22 or 23, wherein the oxidoreductase is cross-linked to the phenothiazine-based compound bound to a polymer.
[25] An electrochemical sensor for detecting or quantifying an analyte, the electrochemical sensor having a working electrode, a counter electrode, and the reagent layer according to any one of items 22 to 24 disposed on the working electrode.
[26] The electrochemical sensor according to item 25, further having a reference electrode.
[27] The electrochemical sensor according to item 25 or 26, further comprising a protective film with which at least the reagent layer is coated.

### Advantageous Effects of Invention

The phenothiazine-based compound bound to a polymer or the phenothiazine-based compound having a hydrophilic moiety introduced therein according to the present disclosure is excellent in functional stability of electron transfer, for example, stability in a living body or a culture environment. Use of such a phenothiazine-based compound bound to a polymer or a phenothiazine-based compound having a hydrophilic moiety introduced therein as a redox polymer or a redox mediator can provide an electrochemical sensor that is suitable for use in measurement over a long period (continuous monitoring) and excellent in durability.

### Brief Description of Drawings

[Figure 1] Figure 1 is a plane view of a sensor according to one embodiment of the present invention. Figure 1(A) shows the whole sensor, and Figure 1(B) shows a tip portion of the sensor in an enlarged manner.
[Figure 2] Figure 2 is a sectional view of the sensor in a specific portion of Figure 1(B). Figure 2(A) is a sectional view taken along the AA line in Figure 1(B).
Figure 2(B) is a sectional view taken along the BB line in Figure 1(B). Figure 2(C) is a sectional view taken along the CC line in Figure 1(B).
[Figure 3] Figure 3 is a top view showing another example of the front side (side having a working electrode and a reference electrode) of the sensor according to one embodiment of the present invention.
[Figure 4] Figure 4 is a sectional view taken along the A-A' section line in Figure 3.
[Figure 5] Figure 5 is a sectional view taken along the B-B' section line in Figure 4.
[Figure 6] Figure 6 is a sectional view taken along the C-C' section line in Figure 4.
[Figure 7] Figure 7 is a plane view of a sensor according to one embodiment of the present invention. Figure 7(A) shows an electrode pattern before film (insulating resist film) formation, and Figure 7(B) shows an electrode pattern after film formation.
[Figure 8] Figure 8 shows PNT compounds of Reference Comparative Examples 1-1 and 1-2, and their absorption spectra in an optical test and cyclic voltammograms in a redox reaction test.
[Figure 9] Figure 9 shows PNT compounds of Reference Examples 1-1 to 1-3, and their absorption spectra in an optical test and cyclic voltammograms in a redox reaction test.
[Figure 10] Figure 10 shows PNT compounds of Reference Examples 1-4 and 1-5, and their absorption spectra in an optical test and cyclic voltammograms in a redox reaction test.
[Figure 11] Figure 11 shows a PNT compound of Example 2-1, and its absorption spectrum in an optical test and cyclic voltammogram in a redox reaction test.
[Figure 12] Figure 12 shows a PNT compound of Example 2-2, and its absorption spectrum in an optical test and cyclic voltammogram in a redox reaction test.
[Figure 13] Figure 13 shows a PNT compound of Example 2-3, and its absorption spectrum in an optical test and cyclic voltammogram in a redox reaction test.
[Figure 14] Figure 14 shows the absorption spectra of polymer-bound PNT compounds of Examples 3-1 to 3-4 in an optical test in PBS.
[Figure 15] Figure 15 shows the absorption spectra of polymer-bound PNT compounds of Examples 3-2 to 3-4 in an optical test in a sodium phosphate buffer solution (pH 6 or pH 8).
[Figure 16] Figure 16 shows the cyclic voltammograms of polymer-bound PNT compounds of Examples 3-2 to 3-4 in an electrochemical response test.
[Figure 17] Figure 17 shows the cyclic voltammograms of polymer-bound PNT compounds of Examples 3-2 to 3-4 in an electrochemical response and durability test.
[Figure 18] Figure 18 is a diagram showing the sensor response of polymer-bound PNT compounds of Examples 3-3 and 3-4 in a sensor durability test.

### Reference Signs List

11: Sensor (probe)
21: Substrate
22: Electrode
22a: Working electrode
22b: Reference electrode
22c: Counter electrode
23: Reagent layer
24: Silver/silver chloride layer
25: Film
X1: Sensor head
X2: Direction of insertion of sensor to living body
X3: Region where counter electrode is not coated with film
X4: Region where sensor head is not coated with film
X5: Region where no reagent layer is formed
X6: Region where neither reagent layer nor electrode (working electrode) is formed (trimmed region)
101: Sensor
111: Insulating substrate
112: Conductive thin film
112a: Working electrode region
112b: Reference electrode region
112c: Counter electrode region
113: Groove
114: Working electrode
115: Reference electrode
116a: Insulating resist film (upper face)
116b: Insulating resist film (lower face)
117: Counter electrode
118: Reagent layer
119: Protective film
121: Sensing portion
122: Terminal portion
201: Sensor
202: Sensing portion
203: Terminal portion
204: Cutout portion
210: Substrate
220: Electrode
221: First working electrode
221a: Exposed region in sensing portion of first working electrode
221b: Exposed region in terminal portion of first working electrode
222: Second working electrode
222a: Exposed region in sensing portion of second working electrode
222b: Exposed region in terminal portion of second working electrode
223: Reference electrode
223a: Exposed region in sensing portion of reference electrode
223b: Exposed region in terminal portion of reference electrode
224: Counter electrode
224a: Exposed region in sensing portion of counter electrode
224b: Exposed region in terminal portion of counter electrode
225: Groove
230: Film (insulating resist film)

### Description of Embodiments

Hereinafter, embodiments of the phenothiazine-based compound, the phenothiazine-based compound having a hydrophilic moiety introduced therein, the phenothiazine-based compound bound to a polymer, the reagent layer, the electrochemical sensor, and the like according to the present invention will be described in more detail.

In the present specification, the following terms respectively have the following meanings.

"Compound (N)" (N is, for example, 1A or 1B) ... a compound represented by the general formula (N).

"Iminium cation" ... a substituent represented by =N⁺R⁹R¹⁰ introduced to the 3-position of a phenothiazine skeleton in the general formula (1A) or the like.

"Cₙ₋ₘ" ... having n to m carbon atoms (in a substituent or the like).

"Reactive N substituent" ... a substituent for a nitrogen atom of an amino group, having a reactive group that can form a covalent bond or a noncovalent bond.

"Nonreactive N substituent" ... a substituent for a nitrogen atom of an amino group, lacking a reactive group that can form a covalent bond or a noncovalent bond.

"Binding substituent" ... a substituent carried by a compound (in the present specification, particularly, a substituent for a phenothiazine skeleton, carried by the phenothiazine-based compound), the substituent having a group reactable with a reactive group carried by a binding partner (in the present specification, particularly, a compound for hydrophilic moiety introduction or a high molecular weight polymer) and thereby enabling the compound to bind to the binding partner.

"Specific binding substituent of phenothiazine-based compound" ... a substituent (R¹, R², and R⁴ to R⁸ in the formulas (1A) and (1B)) for a phenothiazine skeleton, carried by the phenothiazine-based compound, the substituent being capable of binding to a compound for hydrophilic moiety introduction or a high molecular weight polymer.

"Specific reactive group of phenothiazine-based compound (specific binding substituent)" ... a group that is carried by the phenothiazine-based compound and constituted by a portion or the whole of the specific binding substituent, the group being reactable with a first specific reactive group of a compound for hydrophilic moiety introduction or a specific reactive group of a high molecular weight polymer.

"First specific reactive group of compound for hydrophilic moiety introduction" ... a group carried by the compound for hydrophilic moiety introduction, the group being reactable with the specific reactive group of the phenothiazine-based compound (specific binding substituent).

"Second specific reactive group of compound for hydrophilic moiety introduction" ... a group optionally carried by the compound for hydrophilic moiety introduction, the group being reactable with a specific reactive group of a high molecular weight polymer. The compound for hydrophilic moiety introduction is bound to the phenothiazine-based compound and introduced as a hydrophilic moiety to the phenothiazine-based compound, whereby the second specific reactive group of the compound for hydrophilic moiety introduction serves as the specific reactive group of the hydrophilic moiety.

"Specific reactive group of high molecular weight polymer" ... a group carried by a side chain (and end(s)) of the high molecular weight polymer, the group being reactable with the second specific reactive group (carried by the hydrophilic moiety introduced in the phenothiazine-based compound) of the compound for hydrophilic moiety introduction or the specific reactive group carried by the specific binding substituent of the phenothiazine-based compound.

### - Phenothiazine-based compound -

The phenothiazine-based compound according to the present invention can be used as a "redox mediator". The "redox mediator" means an oxidation-reduction material that mediates electron transfer, and refers to, for example, a substance responsible for the transfer of electrons resulting from the redox reaction of an analyte via an oxidoreductase. Various compounds such as ferricyanide and ferrocene are known as redox mediators. The phenothiazine-based compound can be a preferred redox mediator because the compound has a negative redox potential (vs. Ag/AgCl·saturated KCl) (lower than 0 V) and is unsusceptible to impurities against electrochemical measurement contained in samples, for example, oxidizable compounds such as ascorbic acid (vitamin C) or uric acid, which are not analytes, contained in biological samples or medium samples.

The phenothiazine-based compound according to the present invention is a compound represented by the general formula (1A) or (1B). The compound represented by the general formula (1A), i.e., the compound (1A), has a substituent represented by =N⁺R⁹R¹⁰ (iminium cation) introduced at the 3-position of the phenothiazine skeleton. The compound represented by the general formula (1B), i.e., the compound (1B), is in the relationship of a resonant structure with the compound (1A). It should be understood that the compound represented by the general formula (1A) is a compound that can also be represented by the general formula (1B) and the compound represented by the general formula (1B) is a compound that can also be represented by the general formula (1A). In the present specification, items described about the compound represented by the general formula (1A) can also be applied to the compound represented by the general formula (1B), and items described about the compound represented by the general formula (1B) cab also be applied to the compound represented by the general formula (1A), unless otherwise specified.

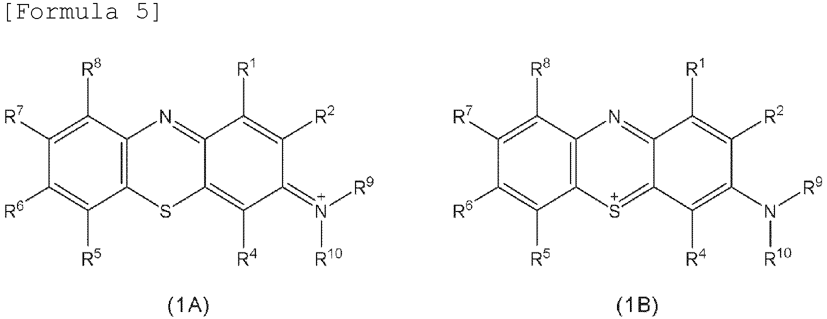

In the general formulas (1A) and (1B), each symbol is defined as follows.

R⁹ and R¹⁰ are both substituents, or one of these moieties is a substituent, and the other moiety is a hydrogen atom (R⁹ and R¹⁰ cannot be simultaneously hydrogen atoms).

The "substituent" as each of R⁹ and R¹⁰ is not particularly limited as long as the phenothiazine-based compound of the present invention can form the compound (1A) having an iminium cation at a predetermined position or the compound (1B) having a resonant structure thereof. Examples thereof include (vi) a "linear or branched saturated or unsaturated hydrocarbon group optionally having a substituent". The hydrocarbon group is preferably, for example, a hydrocarbon group having 1 to 6 carbon atoms (C₁₋₆), particularly preferably a methyl group.

R¹, R², and R⁴ to R⁸ each independently represent any of the following groups or atoms (i) to (viii):
(i) a hydrogen atom;
(ii) a halogen atom;
(iii) a hydroxy group optionally having a substituent;
(iv) a carboxy group optionally having a substituent;
(v) an amino group optionally having a substituent;
(vi) a saturated or unsaturated hydrocarbon group optionally having a substituent (e.g., a C₁₋₁₅ alkyl group, preferably a C₁₋₆ alkyl group);
(vii) an acyl group optionally having a substituent (e.g., a C₁₋₆ acyl group, i.e., a C₁₋₆ alkyl-carbonyl group); and
(viii) a phenyl group optionally having a substituent.

Examples of the substituent that may be carried by each of the substituents (iii) to (viii) include a group including at least one of (a) a halogen atom, (b) a hydroxy group, (c) a carboxy group or an active ester form thereof (e.g., N-hydroxysuccinimide ester (NHS)), (d) an amino group, (e) a linear or branched saturated or unsaturated hydrocarbon group (e.g., a C₁₋₁₅ alkyl group, preferably a C₁₋₆ alkyl group, more preferably a C₁₋₃ alkyl group), (f) an acyl group (e.g., a C₁₋₆ acyl group, preferably a C₁₋₃ acyl group), (g) a guanidino group, (h) a mesyl group, (i) a phenyl group, (j) a thiol group, (k) a formyl group (aldehyde group), (l) an epoxy group, (m) a maleimide group. Specifically, the substituent that may be carried by each of the substituents (iii) to (viii) may be a single group constituted by any of the groups (a) to (m) or may be a group constituted by two or three or more members selected from the groups (a) to (m), for example, (e) a linear or branched saturated or unsaturated hydrocarbon group further substituted by (c) a carboxy group or an active ester form thereof, (d) an amino group, (j) a thiol group, (k) a formyl group, (l) an epoxy group, or (m) a maleimide group. Those skilled in the art can carry out the present invention by selecting a chemically appropriate substituent from (a) to (m) for each of the substituents (iii) to (viii) or by selecting two or three or more chemically appropriate substituents from (a) to (m) and linking these substituents.

The number of substituents that may be carried by each of the substituents (iii) to (viii) is not particularly limited and may be, for example, 1, 2, or 3, or a plurality of substituents may be bonded to one atom (e.g., a carbon atom of an alkyl group or a nitrogen atom of an amino group).

Each of R¹, R², and R⁴ to R⁸ may be a group capable of binding to a specific compound or polymer or may be a group incapable of binding thereto. Each of R¹, R², and R⁴ to R⁸ may be, for example, a substituent for introducing a hydrophilic moiety to a phenothiazine-based compound, the substituent being capable of binding to a compound for hydrophilic moiety introduction in the preparation of a "phenothiazine-based compound having a hydrophilic moiety introduced therein". Each of R¹, R², and R⁴ to R⁸ may be a substituent capable of binding to a high molecular weight polymer. In this case, a "phenothiazine-based compound bound to a polymer" can be prepared in which a phenothiazine-based compound is bound directly to a high molecular weight polymer without the hydrophilic moiety as mentioned above. On the other hand, each of R¹, R², and R⁴ to R⁸ may be a hydrophilic group that contributes to improvement in the hydrophilicity of a phenothiazine-based compound in itself (without binding to a compound for hydrophilic moiety introduction) . Each of R¹, R², and R⁴ to R⁸ may be a group related to performance as a redox mediator.

### <Specific binding substituent of phenothiazine-based compound>

In a preferred embodiment of the phenothiazine-based compound of the present invention, any one or more of R¹, R², and R⁴ to R⁸ is a substituent capable of binding to a compound for hydrophilic moiety introduction or a high molecular weight polymer ("specific binding substituent" of the phenothiazine-based compound according to the present invention). In this embodiment, it is particularly preferred that at least R⁶ be the specific binding substituent. For example, R¹, R², and R⁴ to R⁸ except for the specific binding substituent may all be hydrogen atoms or may each be an atom or a substituent that does not serve as the specific binding substituent, other than hydrogen.

The specific binding substituent is selected from among the substituents (iii) to (viii) shown as specific examples of R¹, R², and R⁴ to R⁸ and is suitable for the binding to a compound for hydrophilic moiety introduction or a high molecular weight polymer, i.e., can have a group reactable under appropriate conditions with a first specific reactive group of the compound for hydrophilic moiety introduction or a specific reactive group of the high molecular weight polymer. Among the substituents (iii) to (viii), a substituent that cannot substantially bind to the compound for hydrophilic moiety introduction or the high molecular weight polymer or has industrial difficulty in binding thereto (e.g., an unsubstituted phenyl group) is excluded from the specific binding substituent of the present invention. Each of the substituents (iii) to (viii) may be a group that is reactable in itself (in a state having no substituent) with a first specific reactive group of the compound for hydrophilic moiety introduction or a specific reactive group of the high molecular weight polymer ("specific reactive group" of the phenothiazine-based compound (specific binding substituent) according to the present invention), or a part or the whole of substituents carried by each of the groups (iii) to (viii) may serve as the specific reactive group. In any of the embodiments, the phenothiazine-based compound (specific binding substituent) can be regarded as "having" the specific reactive group.

### <Specific reactive group of phenothiazine-based compound (specific binding substituent)>

The "specific reactive group of the phenothiazine-based compound (specific binding substituent)" is not particularly limited as long as the group is reactable with a first specific reactive group carried by the compound for hydrophilic moiety introduction for introducing a hydrophilic moiety or a specific reactive group carried by the high molecular weight polymer. The specific reactive group can be selected from various known reactive groups.

The specific reactive group of the phenothiazine-based compound is preferably, for example, "at least one member selected from the group consisting of a carboxy group or an active ester form (e.g., a NHS form) thereof, an amino group, a thiol group, a formyl group (aldehyde group), an epoxy group, and a maleimide group". Such a specific reactive group of the phenothiazine-based compound is preferably a reactive group carried by the specific binding substituent, i.e., each of the groups (iii) to (viii) preferably has the substituent such as (c), (d), (j), (k), (1), or (m) as the specific reactive group. A protective group may be bonded, if necessary, to the specific reactive group of the phenothiazine-based compound and may be deprotected before reaction for the binding of the phenothiazine-based compound to the compound for hydrophilic moiety introduction or the high molecular weight polymer.

In a preferred embodiment of the phenothiazine-based compound of the present invention, any one or more of R¹, R², and R⁴ to R⁸, particularly preferably at least R⁶, is (v) an "amino group optionally having a substituent" described above as the specific binding substituent, i.e., a group represented by the formula: -NR¹¹R¹² (wherein R¹¹ and R¹² each independently represent a hydrogen atom or substituent). In this embodiment, the group represented by-NR¹¹R¹² which serves as the specific binding substituent is an "amino group having a substituent", and it is preferred that one or both, preferably one, of R¹¹ and R¹² be a substituent having the specific reactive group (a reactive N substituent, for example, a C₁₋₆ alkyl group substituted by a carboxy group: - (CH₂)₁₋₆-COOH) and the other moiety be a hydrogen atom or a substituent having no specific reactive group (a nonreactive N substituent, for example, a C₁₋₆ alkyl group). On the other hand, it is also possible that R¹¹ and R¹² are both hydrogen atoms, i.e., -NR¹¹R¹² represents an unsubstituted amino group and the unsubstituted amino group is used as the specific binding substituent (specific reactive group of the phenothiazine-based compound). As one example, the phenothiazine-based compound of the present invention can be represented by the following general formula (1A-1) or (1B-1):

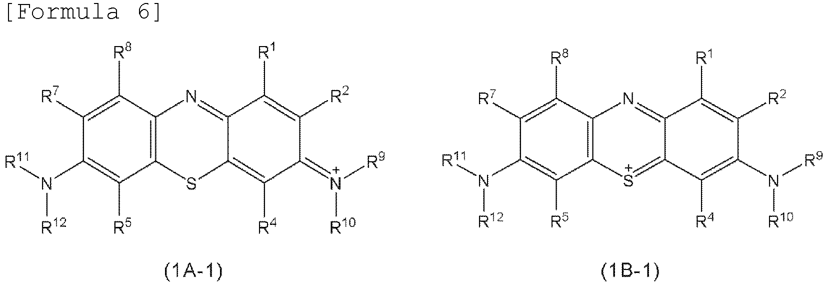

In the general formulas (1A-1) and (1B-1), R¹¹ is a hydrogen atom or a nonreactive N substituent, R¹² is a reactive N substituent, and the other symbols are as defined in the general formulas (1A) and (1B) mentioned above.

The phenothiazine-based compound (including a phenothiazine-based compound having a hydrophilic moiety introduced therein and a phenothiazine-based compound bound to a polymer) according to the present invention may form a salt. The phenothiazine-based compound of the present invention, when containing a positively charged moiety (cation), can form a salt with a negatively charged ion (anion species). The phenothiazine-based compound of the present invention, when containing a negatively charged moiety (anion), can form a salt with a positively charged ion (cation species). Examples of the anion species include halogen ions, ions of compounds containing halogen, hydroxide ions, carboxylate ions, nitrate ions, nitrite ions, formate ions, acetate ions, propionate ions, butyrate ions, hydrogen carbonate ions, dihydrogen phosphate ions, hydrogen sulfate ions, alkylsulfonate ions, hydrogen sulfide ions, hydrogen oxalate ions, cyanate ions, and thiocyanate ions. Examples of the cation species include alkali metal ions such as sodium ions and potassium ions, alkaline earth metal ions such as calcium ions, magnesium ions, and barium ions, metal ions such as aluminum ions, quaternary ammonium cations such as choline ions, ammonium ions, ions of compounds containing an amino group or a substituted amino group, pyridinium ions, and ions of compounds containing a pyridyl group or a substituted pyridyl group.

### - Phenothiazine-based compound having hydrophilic moiety introduced therein -

The phenothiazine-based compound having a hydrophilic moiety introduced therein according to the present invention is a compound having a "hydrophilic moiety" introduced in the phenothiazine-based compound according to the present invention mentioned above, i.e., a compound of the general formula (1A) or (1B) wherein any one or more sites of R¹, R², and R⁴ to R⁸ have a hydrophilic moiety (L) introduced therein. Such a compound can be represented by the general formula (2A) or (2B).

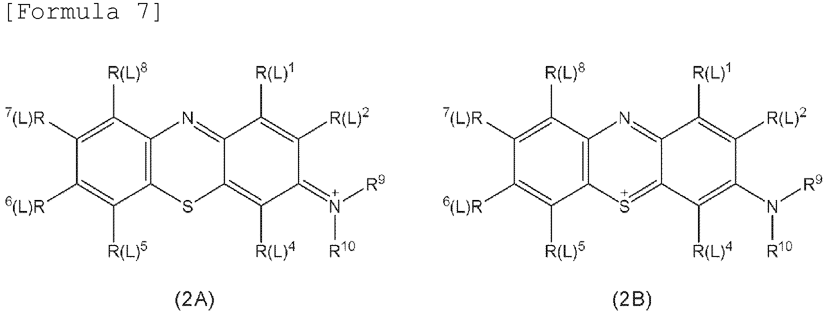

In the general formulas (2A) and (2B), each symbol is defined as follows.

R⁹ and R¹⁰ are both substituents, or one of these moieties is a substituent, and the other moiety is a hydrogen atom (as defined in the general formulas (1A) and (1B)).

R(L)¹, R(L)², and R(L)⁴ to R(L)⁸ are each independently [A] a hydroxy group optionally having a substituent, a carboxy group optionally having a substituent, an amino group optionally having a substituent, a linear or branched saturated or unsaturated hydrocarbon group having 1 to 6 carbon atoms and optionally having a substituent, an acyl group optionally having a substituent, or a phenyl group optionally having a substituent, [B] a group formed by the binding of a compound for hydrophilic moiety introduction to the binding substituent selected from [A], or [C] a hydrogen atom or a halogen atom. Specifically, the hydroxy group optionally having a substituent, the carboxy group optionally having a substituent, the amino group optionally having a substituent, the linear or branched saturated or unsaturated hydrocarbon group having 1 to 6 carbon atoms and optionally having a substituent, the acyl group optionally having a substituent, or the phenyl group optionally having a substituent may be further bound to a compound for hydrophilic moiety introduction (i.e., may have a hydrophilic moiety introduced therein) or may not be bound thereto (i.e., may not have a hydrophilic moiety introduced therein). However, at least one of R(L)¹, R(L)², and R(L)⁴ to R(L)⁸ is the group [B], i.e., a group formed by the binding of a compound for hydrophilic moiety introduction to the group [A] (i.e., a group having a hydrophilic moiety introduced therein). When two or more of R(L)¹, R(L)², and R(L)⁴ to R(L)⁸ are such groups, their respective hydrophilic moieties may be the same or different.

In a preferred embodiment of the phenothiazine-based compound having a hydrophilic moiety introduced therein according to the present invention, at least R(L)⁶ has a hydrophilic moiety introduced therein, and if necessary, further one or more of R(L)¹, R(L)², R(L)⁴, R(L)⁵, R(L)⁷, and R(L)⁸ may have a hydrophilic moiety introduced therein. In this embodiment, the phenothiazine-based compound having a hydrophilic moiety introduced therein is of the general formula (2A) or (2B) wherein at least R(L)⁶ corresponds to a group formed by the binding of a compound for hydrophilic moiety introduction to a specific binding substituent for hydrophilic moiety introduction having a specific reactive group for hydrophilic moiety introduction. The details of the "specific binding substituent" and the "specific reactive group" are as described in the present specification in relation to the phenothiazine-based compound according to the present invention. As one example, the phenothiazine-based compound having a hydrophilic moiety introduced therein according to the present invention in which the hydrophilic moiety is introduced at the site of R⁶ can be represented by the following general formula (2A-1) or (2B-1):

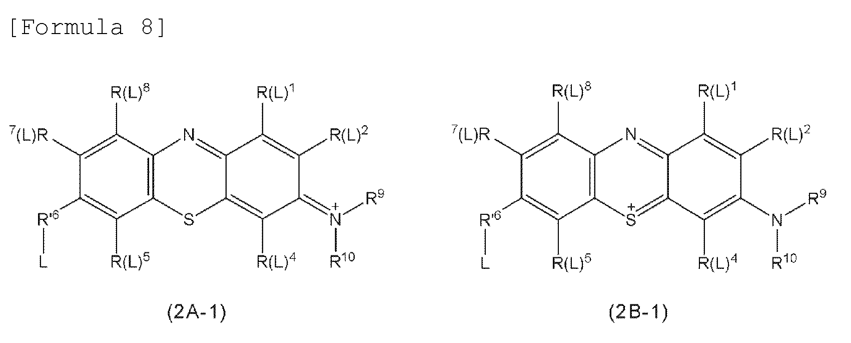

In the general formulas (2A-1) and (2B-1), R'⁶ represents a group derived from the specific binding substituent, L represents the hydrophilic moiety, and the other symbols are each as defined in the general formulas (2A) and (2B).

In a preferred embodiment of the phenothiazine-based compound having a hydrophilic moiety introduced therein according to the present invention, any one or more of R(L)¹, R(L)², and R(L)⁴ to R(L)⁸, particularly preferably at least R(L)⁶, is a group formed by the binding of a compound for hydrophilic moiety introduction to a specific binding substituent represented by the formula: -NR¹¹R¹². The details of the formula: -NR¹¹R¹² are described in the present specification in relation to the phenothiazine-based compound of the present invention. As one example, the phenothiazine-based compound having a hydrophilic moiety introduced therein according to the present invention in which R(L)⁶ is a group formed by the binding of a compound for hydrophilic moiety introduction to a specific binding substituent represented by-NR¹¹R¹² can be represented by the following general formula (2A-2) or (2B-2):

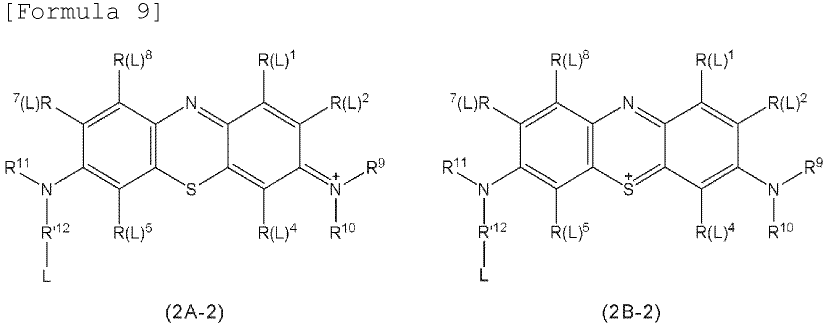

In the general formulas (2A-2) and (2B-2), R¹¹ is a hydrogen atom or a nonreactive N substituent, R'¹² is a group derived from a reactive N substituent, L is the hydrophilic moiety derived from the compound for hydrophilic moiety introduction, and the other symbols are as defined in the general formulas (2A-1) and (2B-1) mentioned above.

### <Hydrophilic moiety>

In the phenothiazine-based compound having a hydrophilic moiety introduced therein according to the present invention, the "hydrophilic moiety" is a moiety (chemical structure) for imparting "hydrophilicity" to a phenothiazine-based compound and may be, depending on an embodiment, a moiety suitable for linking a high molecular weight polymer and a phenothiazine-based compound. The hydrophilic moiety at least partially (e.g., at a first end or in the vicinity thereof) has a bond structure that is derived from a first specific reactive group carried by a compound for hydrophilic moiety introduction, and formed through reaction with the specific reactive group of the phenothiazine-based compound (specific binding substituent). The hydrophilic moiety may have, at a site different therefrom (e.g., at a second end or in the vicinity thereof), a second specific reactive group carried by the compound for hydrophilic moiety introduction, as a group reactable with a specific reactive group of a high molecular weight polymer, or may not have such a group reactable with a specific reactive group of a high molecular weight polymer.

As used herein, the "hydrophilicity" refers to a nature that has high affinity for water and permits dissolution or blending to the degree that a goal can be achieved in water or other polar solvents, for example, a solvent for reacting the phenothiazine-based compound having a hydrophilic moiety introduced therein with a high molecular weight polymer in synthesizing a phenothiazine-based compound bound to a polymer, or a solvent for dissolving the phenothiazine-based compound bound to a polymer (redox polymer) in forming a predetermined layer in an electrochemical sensor. Examples of the polar solvent include methanol, ethanol, 1-propanol, 2-propanol, 1-butanol, 2-butanol, 2-methyl-1-propanol, 2-methyl-2-propanol, formic acid, acetic acid, tetrahydrofuran, acetone, dioxane, methyl ethyl ketone, ethyl acetate, acetonitrile, dimethylformamide, and dimethyl sulfoxide.

The hydrophilic moiety according to the present invention is not particularly limited and can be selected from various hydrophilic moieties. Examples of the hydrophilic moiety having a relatively small size (molecular length, etc.) in terms of a chemical structure include functional groups themselves referred to as so-called "hydrophilic groups", such as a hydroxy group, an amino group, a carboxy group, a sulfo group, and a quaternary ammonium cation, and groups that are derived from a molecule containing such a hydrophilic group and have hydrophilicity. On the other hand, examples of the hydrophilic moiety having a relatively large size in terms of a chemical structure include structures that are referred to as so-called "linker groups (moieties)" and have hydrophilicity (in the present specification, such a hydrophilic moiety is referred to as a "linker-like hydrophilic moiety").

### <Linker-like hydrophilic moiety>

The linker-like hydrophilic moiety is generally a site having a chain structure where a moiety except for reactive groups before reaction located at both ends or a bond structure after reaction is composed mainly of carbon atoms and may contain at least one heteroatom selected from the group consisting of a nitrogen atom, an oxygen atom, and a sulfur atom, in other words, a chain structure that may contain a heteroatom-containing bond such as an ether bond, a thioether bond, or an amide bond in the middle. The "chain structure" may be linear or branched and is preferably linear from the viewpoint of hydrophilicity. The "chain structure" preferably does not contain any ring structure derived from a cyclic compound (aromatic hydrocarbon ring, nonaromatic hydrocarbon ring, aromatic heterocyclic ring, nonaromatic heterocyclic ring, etc.) from the viewpoint of hydrophilicity and may contain one or more ring structures as long as the desired hydrophilicity is obtained.

In a preferred embodiment of the present invention, the linker-like hydrophilic moiety comprises an oxyethylene chain represented by the formula: -(OC₂H₄)_{q}-. In the formula, q represents, for example, an integer of 1 to 80, preferably an integer of 3 to 36. An oxyethylene chain represented by the formula wherein q is a large number to some extent is generally called "polyoxyethylene (PEG chain)" or the like.

In one embodiment of the present invention, the linker-like hydrophilic moiety comprises a hydrocarbon chain represented by the formula: -(CH₂)ₚ-. In one embodiment of the present invention, the backbone of the linker-like hydrophilic moiety comprises both of a hydrocarbon chain represented by the formula: -(CH₂)ₚ- and an oxyethylene chain represented by the formula: - (OC₂H₄)_{q}-. In the formula representing a hydrocarbon chain, p can be appropriately adjusted so as to achieve a balance with q in the formula representing an oxyethylene chain in consideration of the hydrophilicity of the linker-like hydrophilic moiety.

### <Specific reactive group of hydrophilic moiety (second specific reactive group of compound for hydrophilic moiety introduction)>

The specific reactive group of the hydrophilic moiety, i.e., the reactive group for binding to a high molecular weight polymer, which may be carried by the compound for hydrophilic moiety introduction ("second specific reactive group" of the compound for hydrophilic moiety introduction) is not particularly limited as long as the group is reactable with a specific reactive group of a high molecular weight polymer. The specific reactive group can be selected from various known reactive groups. Such a specific reactive group of the hydrophilic moiety (second specific reactive group of the compound for hydrophilic moiety introduction) is preferably, for example, "at least one member selected from the group consisting of a carboxy group or an active ester form (e.g., NHS form) thereof, an amino group, a thiol group, a formyl group (aldehyde group), an epoxy group, and a maleimide group". A protective group may be bonded, if necessary, to the reactive group for polymer binding and may be deprotected before reaction for the binding of the phenothiazine-based compound having a hydrophilic moiety introduced therein or the high molecular weight polymer.

### <General reactive group>

The predetermined reactive groups according to the present invention, i.e., the specific reactive group of the phenothiazine-based compound, the first specific reactive group of the compound for hydrophilic moiety introduction or the specific reactive group of the high molecular weight polymer which binds thereto, the second specific reactive group of the compound for hydrophilic moiety introduction (hydrophilic moiety), and the specific reactive group of the high molecular weight polymer which binds thereto can be variously combined by adopting their respective various known reactive groups. The predetermined reactive groups according to the present invention may be covalently bound to each other or may be noncovalently (e.g., through electrostatic interaction) bound to each other, and preferably, are covalently bound to each other, for example, from the viewpoint of binding stability.

The predetermined reactive groups according to the present invention are each independently preferably at least one member selected from the "group consisting of a carboxy group or an active ester form thereof, an amino group, a thiol group, a formyl group (aldehyde group), an epoxy group, and a maleimide group" (in the present specification, referred to as the "preferred group of reactive groups"). Table 1 shows groups with which the reactive groups included in the preferred group of reactive groups can react respectively. In the case of selecting a reactive group included in the preferred group of reactive groups as one of the predetermined reactive groups according to the present invention, the partner group to be reacted therewith may be another reactive group included in the preferred group of reactive groups (underlined in the table) or may be a reactive group excluded from the preferred group of reactive groups (not underlined or not included in the table).

**[Table 1]**

| Reactive group A | Reactive group B | Formed bond, etc. |
|---|---|---|
| Carboxy group R^{a}-COOH or active ester form thereof | Amino group R^{b}-NH₂ | Amide bond R^{a}-CO-NH-R^{b} |
| | Alcoholic or phenolic hydroxy group R^{b}-OH | Ester bond R^{a}-CO-O-R^{b} |
| | Thiol group R^{b}-SH | Thioester bond R^{a}-CO-S-R^{b} |
| | Alkyl halide group R^{b}-X | Ester bond R^{a}-CO-O-R^{b} |
| Amino group R^{a}-NH₂ | Carboxy group or active ester form thereof R^{b}-COOH | Amide bond R^{a}-NH-CO-R^{b} |
| | Acyl azide group | Amide bond |
| | Acyl halide group | Amide bond |
| | Isocyanate group | Urea bond |
| | Isothiocyanate group | Thiourea bond |
| | Imide ester | Amidine bond |
| | Epoxy group | Amide bond R^{a}-NH-CO-R^{b} |
| | 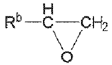 | |
| | Alkyl halide group | Alkylamide bond |
| | Sulfonyl halide group | Sulfonamide bond |
| Thiol group R^{a}-SH | Carboxy group or active ester form thereof R^{b}-COOH | Thioester bond R^{a}-S-CO-R^{b} |
| | Maleimide group | Thioether bond |
| | 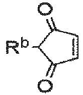 | 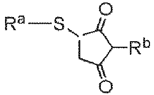 |
| | Epoxy group | Thioether bond |
| | 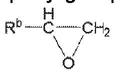 | |
| | Alkyl halide group | Thioether bond |
| | Alkyl sulfonate group | Thioether bond |
| | Aziridine group | Thioether bond |
| Formyl group (aldehyde group) R^{a}-CHO | Alkoxyamino group R^{b}-O-NH₂ | Oxime bond R^{a}-CH=N-O-R^{b} |
| | Amino group R^{b}-NH₂ | Shiff base R^{a}-CH=N-R^{b} |
| | Hydrazine group R^{b1}R^{b2}N-NH₂ | Hydrazone bond R^{a}-CH=N-NR^{b1}R^{b2} |

In a preferred embodiment of the present invention, one of the "second specific reactive group" of the compound for hydrophilic moiety introduction (hydrophilic moiety) or the "specific reactive group of the phenothiazine-based compound" and the "specific reactive group of the high molecular weight polymer" is the "reactive group A" of Table 1, and the other group is the "reactive group B". Likewise, in a preferred embodiment of the present invention, one of the "first specific reactive group" of the compound for hydrophilic moiety introduction and the "specific reactive group of the phenothiazine-based compound" is the "reactive group A" of Table 1, and the other group is the "reactive group B" .

In a more preferred embodiment of the present invention, one of the "second specific reactive group" of the compound for hydrophilic moiety introduction (hydrophilic moiety) or the "specific reactive group of the phenothiazine-based compound" and the "specific reactive group of the high molecular weight polymer" is an amino group, and the other group is a carboxy group or an active ester form thereof. Likewise, in a more preferred embodiment of the present invention, one of the "first specific reactive group" of the compound for hydrophilic moiety introduction and the "specific reactive group of the phenothiazine-based compound" is an amino group, and the other group is a carboxy group or an active ester form thereof. For example, in the case of using a phenothiazine-based compound having an active ester form of carboxylic acid as the specific reactive group; a compound for hydrophilic moiety introduction having an amino group as the first specific reactive group and an active ester form of carboxylic acid (e.g., carboxylic acid NHS ester form) as the second specific reactive group; and a high molecular weight polymer having an amino group as the specific reactive group, first, the active ester form of carboxylic acid of the phenothiazine-based compound is reacted with the amino group of the compound for hydrophilic moiety introduction to obtain a phenothiazine-based compound having a hydrophilic moiety introduced therein in which these components are bound to each other through a first amide bond, and the active ester form of carboxylic acid of the obtained phenothiazine-based compound having a hydrophilic moiety introduced therein is reacted with the amino group of the high molecular weight polymer to obtain a phenothiazine-based compound bound to a polymer in which these components are bound to each other through a second amide bond.

### <Compound for hydrophilic moiety introduction>

The "compound for hydrophilic moiety introduction" according to the present invention is a compound for binding to the specific binding substituent carried by the phenothiazine-based compound in order to introduce (additionally form) the "hydrophilic moiety" as mentioned above. For example, a compound having a structure that corresponds to the linker-like hydrophilic moiety described in relation to the hydrophilic moiety and a first specific reactive group for binding to the phenothiazine-based compound in a portion thereof (e.g., at a first end or in the vicinity thereof) and optionally having a second specific reactive group for binding to a high molecular weight polymer in a moiety different therefrom (e.g., at a second end or in the vicinity thereof) can be used as the compound for hydrophilic moiety introduction. Alternatively, a compound having a smaller size (molecular weight) that can form the hydrophilic moiety may be used as the compound for hydrophilic moiety introduction. Such a compound for hydrophilic moiety introduction is not particularly limited and can be selected from various known compounds for hydrophilic moiety introductions.

### <First specific reactive group of compound for hydrophilic moiety introduction>

The first specific reactive group carried by the compound for hydrophilic moiety introduction is not particularly limited as long as the group is reactable with the specific reactive group of the phenothiazine-based compound. The first specific reactive group can be selected from various known reactive groups. Such a first specific reactive group of the compound for hydrophilic moiety introduction is preferably, for example, "at least one member selected from the group consisting of a carboxy group or an active ester form (e.g., NHS form) thereof, an amino group, a thiol group, a formyl group (aldehyde group), an epoxy group, and a maleimide group". A protective group may be bonded, if necessary, to the first specific reactive group of the compound for hydrophilic moiety introduction and may be deprotected before reaction for the binding of the compound for hydrophilic moiety introduction to the phenothiazine-based compound. The second specific reactive group that may be carried by the compound for hydrophilic moiety introduction is a reactive group that corresponds to the specific reactive group that may be carried by the hydrophilic moiety, and its technical item is as mentioned above.

### <Method for producing phenothiazine-based compound having hydrophilic moiety introduced therein>

The phenothiazine-based compound having a hydrophilic moiety introduced therein can be synthesized by reacting a phenothiazine-based compound having the desired chemical structure with a compound for hydrophilic moiety introduction having the desired chemical structure under appropriate conditions (temperature, time, adjuvant, etc.). The synthesized phenothiazine-based compound having a hydrophilic moiety introduced therein may be purified, if necessary, by gel filtration chromatography, ultrafiltration, or the like.

The phenothiazine-based compound having a hydrophilic moiety introduced therein can be produced and used as various types depending on the respective structures of R(L)¹, R(L)² and R(L)⁴ to R(L)⁸ as well as R⁹ and R¹⁰ in the general formulas (2A) and (2B). In the present invention, any one type of phenothiazine-based compound having a hydrophilic moiety introduced therein may be used singly or two or more types thereof may be used in combination, for example, as a starting material for synthesizing a phenothiazine-based compound bound to a polymer as mentioned later.

### - Phenothiazine-based compound bound to polymer -

The phenothiazine-based compound bound to a polymer according to the present invention is a phenothiazine-based compound having a structure where the phenothiazine-based compound or the phenothiazine-based compound having a hydrophilic moiety introduced therein according to the present invention mentioned above is bound to a high molecular weight polymer, i.e., a structure where the phenothiazine-based compound of the present invention is bound to a high molecular weight polymer via or without a hydrophilic moiety. In other words, the phenothiazine-based compound bound to a polymer according to the present invention is a high molecular weight polymer that can be used as a redox polymer wherein the phenothiazine-based compound or the phenothiazine-based compound having a hydrophilic moiety introduced therein is bound to the high molecular weight polymer. The binding of the phenothiazine-based compound or the phenothiazine-based compound having a hydrophilic moiety introduced therein as a redox mediator to a high molecular weight polymer can prevent or suppress leakage from a protective film. The phenothiazine-based compound bound to a polymer can be represented by, for example, the general formula (3A) or (3B).

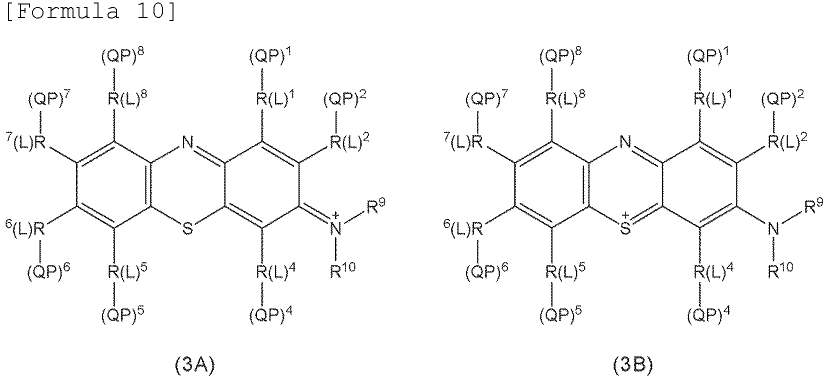

In the general formulas (3A) and (3B), each symbol is defined as follows.

R⁹ and R¹⁰ are both substituents, or one of these moieties is a substituent, and the other moiety is a hydrogen atom (as defined in the general formulas (1A) and (1B) as well as (2A) and (2B)).

R(L)¹, R(L)², and R(L)⁴ to R(L)⁸ are each independently [A] a hydroxy group optionally having a substituent, a carboxy group optionally having a substituent, an amino group optionally having a substituent, a linear or branched saturated or unsaturated hydrocarbon group having 1 to 6 carbon atoms and optionally having a substituent, an acyl group optionally having a substituent, or a phenyl group optionally having a substituent, [B] a group formed by the binding of a compound for hydrophilic moiety introduction to the binding substituent selected from [A], [C] a hydrogen atom or a halogen atom, or [D] a group derived from either the binding substituent selected from [A] or the group [B].

(QP)¹, (QP)², and (QP)⁴ to (QP)⁸ are each independently a polymeric structure derived from a high molecular weight polymer which is present when their corresponding R(L)¹, R(L)², and R(L)⁴ to R(L)⁸ are the group [D]. Specifically, the high molecular weight polymer-derived polymeric structures represented by (QP)¹, (QP)², and (QP)⁴ to (QP)⁸ may be bonded to R(L)¹, R(L)², and R(L)⁴ to R(L)⁸ having a hydrophilic moiety introduced therein or may be bonded to R(L)¹, R(L)², and R(L)⁴ to R(L)⁸ having no hydrophilic moiety introduced therein, i.e., the specific binding substituents themselves represented by R¹, R² and R⁴ to R⁸.

However, at least one of (QP)¹, (QP)², and (QP)⁴ to (QP)⁸ is present. When two or more of (QP)¹, (QP)², and (QP)⁴ to (QP)⁸ are present, their high molecular weight polymers may be the same or may be different (i.e., molecules of a plurality of high molecular weight polymers may be cross-linked through the phenothiazine-based compound having a hydrophilic moiety introduced therein) . When two or more of R(L)¹, R(L)², and R(L)⁴ to R(L)⁸ respectively corresponding to two or more of (QP)¹, (QP)², and (QP)⁴ to (QP)⁸ are specific binding substituents having a hydrophilic moiety introduced therein, their respective hydrophilic moieties may be the same or different.

In a preferred embodiment of the phenothiazine-based compound bound to a polymer according to the present invention, at least one of R(L)¹, R(L)², and R(L)⁴ to R(L)⁸ is a group derived from either a specific binding substituent having at least one specific reactive group selected from the group consisting of a carboxy group or an active ester form thereof, an amino group, a thiol group, a formyl group, an epoxy group, and a maleimide group, or a group formed by the binding of a compound for hydrophilic moiety introduction to the specific binding substituent.

In a preferred embodiment of the phenothiazine-based compound bound to a polymer according to the present invention, at least one of R(L)¹, R(L)², and R(L)⁴ to R(L)⁸ is a group derived from a group formed by the binding of a compound for hydrophilic moiety introduction having at least one specific reactive group selected from the group consisting of a carboxy group or an active ester form thereof, an amino group, a thiol group, a formyl group, an epoxy group, and a maleimide group to the specific binding substituent.

In a preferred embodiment of the phenothiazine-based compound bound to a polymer according to the present invention, the specific binding substituent of the phenothiazine-based compound is bound to a high molecular weight polymer via or without a hydrophilic moiety at least in R(L)⁶(QP)⁶ and may be further bound, if necessary, to a high molecular weight polymer via or without a hydrophilic moiety in one or more of R(L)¹(QP)¹, R(L)²(QP)², R(L)⁴(QP)⁴, R(L)⁵(QP)⁵, R(L)⁷(QP)⁷, and R(L)⁸(QP)⁸. In this embodiment, the phenothiazine-based compound bound to a polymer corresponds to the case where at least R(L)⁶ in the general formula (3A) and (3B) is a group derived from either the specific binding substituent or a group derived from the specific binding substituent having a hydrophilic moiety introduced therein. The details of the "specific binding substituent", the "specific reactive group", and the "hydrophilic moiety" are as described in the present specification in relation to the phenothiazine-based compound and the phenothiazine-based compound having a hydrophilic moiety introduced therein according to the present invention. As one example, the phenothiazine-based compound bound to a polymer according to the present invention in which a compound for hydrophilic moiety introduction is bound to the specific binding substituent in R(L)⁶(QP)⁶ and a high molecular weight polymer is bound via the hydrophilic moiety introduced by the binding can be represented by the following general formula (3A-1) or (3B-1):

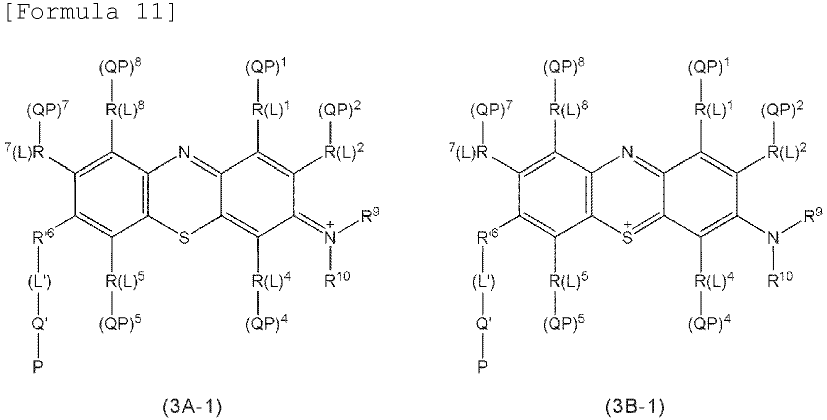

In the general formulas (3A-1) and (3B-1), R'⁶ is a group derived from a specific substituent for binding (R), (L') is optionally present and is a group derived from a compound for hydrophilic moiety introduction (L), Q' is a group derived from a reactive group (Q) of a high molecular weight polymer, P is a backbone of the high molecular weight polymer, and the other symbols are as defined in the general formula (3A) and (3B). The details of the backbone (P) and the reactive group (Q) of the high molecular weight polymer and the high molecular weight polymer having these are mentioned later.

In a preferred embodiment of the phenothiazine-based compound bound to a polymer according to the present invention, any one or more of R(L)¹, R(L)², and R(L)⁴ to R(L)⁸, particularly preferably at least R(L)⁶, is a group derived from either a specific binding substituent which is an amino group having a substituent, the binding substituent being represented by the formula: -NR¹¹R¹², or a group formed by the binding of a compound for hydrophilic moiety introduction to the specific binding substituent. The details of the formula: -NR¹¹R¹² are as described in the present specification in relation to the phenothiazine-based compound of the present invention. As one example, the phenothiazine-based compound bound to a polymer according to the present invention in which R(L)⁶ is a group represented by -NR¹¹R¹² as a specific binding substituent to which a compound for hydrophilic moiety introduction may or may not be bound (i.e., a hydrophilic moiety may or may not be introduced), and a high molecular weight polymer is further bound thereto via or without a hydrophilic moiety can be represented by the following general formula (3A-2) or (3B-2):

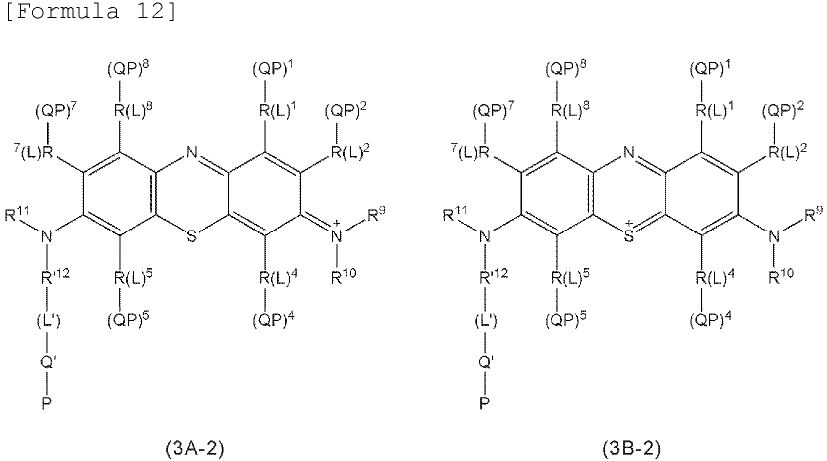

In the general formulas (3A-2) and (3B-2), R¹¹ is a hydrogen atom or a nonreactive N substituent, R'¹² is a group derived from a reactive N substituent, and the other symbols are as defined in the general formulas (3A-1) and (3B-1) mentioned above.

The general formulas (3A-1) and (3B-1), (3A-2) and (3B-2), etc. apparently represent that one molecule of the phenothiazine-based compound (having a hydrophilic moiety introduced therein) is bound to the backbone (P) of one high molecular weight polymer via a group (Q') derived from one reactive group (Q). However, the phenothiazine-based compound bound to a polymer according to the present invention should not be interpreted in such a limited manner and should be interpreted such that a plurality of (many) phenothiazine-based compounds (having a hydrophilic moiety introduced therein) are bound to the backbone of one high molecular weight polymer via a plurality of (many) side chains (and ends) of the high molecular weight polymer.

In the present specification, as mentioned later, a method for producing a redox polymer, comprising the step of reacting the phenothiazine-based compound (having a hydrophilic moiety introduced therein) with a high molecular weight polymer produces a high molecular weight polymer composition having a distribution as to the number of phenothiazine-based compounds (having a hydrophilic moiety introduced therein) bound to one molecule of a high molecular weight polymer. The pattern of the distribution as to the number of phenothiazine-based compounds (having a hydrophilic moiety introduced therein) bound, carried by the high molecular weight polymer composition or the (average) number of phenothiazine-based compounds (having a hydrophilic moiety introduced therein) bound to one molecule of a high molecular weight polymer is not particularly limited and can be appropriately adjusted in consideration of application, performance, etc. and depending on a starting material (a high molecular weight polymer or a monomer constituting the polymer), reaction conditions, etc. in producing a redox polymer. For example, (1) the number of reactive groups contained in one molecule of a monomer for use as a starting material for the high molecular weight polymer is increased or decreased, or (2) the proportion of groups to be actually reacted with a reactive group contained in the phenothiazine-based compound (having a hydrophilic moiety introduced therein), among reactive groups contained in the whole monomer is adjusted depending on reaction conditions, whereby the (average) number of phenothiazine-based compounds (having a hydrophilic moiety introduced therein) bound to one molecule of a high molecular weight polymer can be increased or decreased or the pattern of the distribution can be changed so that a high molecular weight redox polymer (composition) having the desired properties can be produced.

Typically, the phenothiazine-based compound bound to a polymer according to the present invention for use as a redox polymer preferably has hydrophilicity. The details of the "hydrophilicity" are as mentioned above in relation to the hydrophilic moiety. For the phenothiazine-based compound bound to a polymer, it is preferred that the hydrophilic moiety or the high molecular weight polymer, or both, impart hydrophilicity to the phenothiazine-based compound bound to a polymer. For the phenothiazine-based compound bound to a polymer according to the present invention, it is preferred that the high molecular weight polymer be bound to the phenothiazine-based compound, for example, via a hydrophilic moiety (as one example, a PEG chain or other linker-like hydrophilic moieties), and it is more preferred that the high molecular weight polymer itself also have hydrophilicity.

### <High molecular polymer>

The high molecular weight polymer is a polymer having a given level or higher of a molecular weight (weight-average molecular weight) and may have a structure that can bind to the phenothiazine-based compound (having a hydrophilic moiety introduced therein) as a redox mediator and support it, i.e., may have a reactive group ("specific reactive group" of the high molecular weight polymer according to the present invention) reactable with the specific reactive group carried by the hydrophilic moiety of the phenothiazine-based compound having a hydrophilic moiety introduced therein or the specific reactive group carried by the specific binding substituent of the phenothiazine-based compound, and be capable of forming a reagent layer on a working electrode. The type (backbone and side chain structures, etc.) of the high molecular weight polymer is not particularly limited, and any one type may be used singly or two or more types may be used in combination.

The molecular weight (weight-average molecular weight) of the high molecular weight polymer refers to a weight-average molecular weight of usually 10000 or larger, preferably 50000 or larger, more preferably 100000 or larger. The upper limit of the weight-average molecular weight of the high molecular weight polymer is not particularly limited and is usually less than 10000000, preferably less than 1000000.

The weight-average molecular weight or the molecular weight distribution of the high molecular weight polymer may be measured by a known approach depending on the type of the high molecular weight polymer. For example, gel permeation chromatography (GPC) or SDS-polyacrylamide gel electrophoresis (SDS-PAGE) (if the high molecular weight polymer is a protein) can be used. When a commercially available product is purchased and used as the high molecular weight polymer, a numeric value shown (e.g., as "Mw" or "M.W.") in its catalog or the like can be regarded as the weight-average molecular weight.

The backbone of the high molecular weight polymer is generally a chain structure that is composed mainly of carbon atoms and may contain at least one heteroatom selected from the group consisting of a nitrogen atom, an oxygen atom, and a sulfur atom, in other words, a chain structure that may contain a heteroatom-containing bond such as an ether bond, a thioether bond, or an amide bond. The high molecular weight polymer appropriately has, at a side chain, a specific reactive group for binding to the phenothiazine-based compound (having a hydrophilic moiety introduced therein) and may further have a specific reactive group at one or both of the ends of the backbone, from the viewpoint that one molecule of the high molecular polymer can support a plurality of (many) phenothiazine-based compounds (having a hydrophilic moiety introduced therein). Various polymers are known as the high molecular weight polymer having such a backbone and a specific reactive group (side chain and/or end(s)), and the high molecular weight polymer may be synthesized using an appropriate monomer. The high molecular weight polymer may be any of a homopolymer, a copolymer, and a polymer obtained by the binding and/or mixing thereof and may be a random polymer or a block polymer.

### <Specific reactive group of high molecular weight polymer>

The specific reactive group carried by the high molecular weight polymer according to the present invention can be reactable with the specific reactive group of a hydrophilic moiety for binding to the phenothiazine-based compound having a hydrophilic moiety introduced therein (i.e., for binding to the phenothiazine-based compound via a hydrophilic moiety) and can be reactable with the specific reactive group carried by the phenothiazine-based compound itself for binding to the phenothiazine-based compound without a hydrophilic moiety. Such a reactive group is not particularly limited and can be selected from various known reactive groups. The specific reactive group of the high molecular weight polymer is preferably, for example, "at least one member selected from the group consisting of a carboxy group or an active ester form (e.g., NHS form) thereof, an amino group, a thiol group, a formyl group (aldehyde group), a thiol group, and a maleimide group". A protective group may be bonded, if necessary, to the specific reactive group of the high molecular weight polymer and may be deprotected before reaction for the binding of the high molecular weight polymer to the phenothiazine-based compound (having a hydrophilic moiety introduced therein).

The specific reactive group of the high molecular weight polymer may be a group originally carried by a monomer used in the synthesis of the high molecular weight polymer or may be introduced later, for example, may be added by further introducing a reactive substituent to a reactive group originally carried by a monomer for the synthesis of the high molecular weight polymer. In the latter embodiment, the "reactive substituent" is the same as the group containing at least one of (a) to (m), which may be carried by the substituents (iii) to (viii), mentioned above in relation to the phenothiazine-based compound. In both the embodiments described above, the high molecular weight polymer can be regarded as "having" the specific reactive group.

Specific examples of the high molecular weight polymer include amino acid-based polymers, imine-based polymers, and ethylene-based polymers having the specific reactive group at a side chain (and end(s)). The high molecular weight polymer also encompasses a protein or a polypeptide having a natural amino acid sequence or a modified (such a modification includes substitution, deletion, addition, or the like) amino acid sequence, which can be regarded as a polymer with amino acids as monomers (on the proviso that such a protein or polypeptide is conceptually discriminated from an artificially synthesized amino acid-based polymer). The high molecular weight polymer also encompasses a polysaccharide-based polymer having an appropriate reactive group or having an appropriate reactive group introduced therein.

Examples of the ethylene-based polymer include homopolymers and copolymers synthesized from one or two or more monomers selected from monomers containing a radically polymerizable ethylenic carbon-carbon double bond, for example, a vinyl group (CH₂=CH-), an allyl group (CH₂=CH-CH₂-), an acryloyl group (CH₂=CH-C(O)-), or a methacryloyl group (CH₂=C(CH₃)-C(O)-), and their modified forms (e.g., vinyl alcohol forms obtained by the saponification of a unit derived from vinyl acetate, and forms treated so as to impart hydrophilicity thereto). Examples of the monomer containing an ethylenic carbon-carbon double bond include ethylene, propylene, butadiene, isobutene, tetrafluoroethylene, vinyl alcohol, vinyl acetate, vinyl chloride, vinylidene chloride, styrene, methylstyrene, allylamine, diallylamine, diallyldimethylammonium chloride, acrylic acid, methacrylic acid, methyl acrylate, methyl methacrylate, butyl acrylate, butyl methacrylate, hydroxyethyl methacrylate, and acrylonitrile. Examples of the ethylene-based polymer include polyallylamine hydrochloride, allylamine hydrochloride-diallylamine hydrochloride copolymers, and allylamine-diallyldimethylammonium chloride copolymers. Preferred examples of the ethylene-based polymer also include (meth)acrylic polymers such as copolymers of methyl methacrylate and hydroxyethyl methacrylate, copolymers of butyl methacrylate and hydroxyethyl methacrylate, and poly(2-methacryloyloxyethyl phosphorylcholine-co-n-butyl methacrylate), and polyester-based polymers such as polyethylene terephthalate, which are generally known as biocompatible polymers. Preferred examples of the ethylene-based polymer also include (meth)acrylic polymers having a quaternary ammonium cation, an amino group, or the like at a side chain (and end(s)) .

Examples of the imine-based polymer include poly(ethylenimine). The poly (ethylenimine) has a structure such as -(CH₂)₂-NH₂, -(CH₂)₂-NH-(CH₂)₂-NH₂, or - (CH₂)₂-N((CH₂)₂-NH₂)₂ at a side chain, and an amino group: -NH₂ in the structure serves as the specific reactive group of the high molecular weight polymer and can bind to the phenothiazine-based compound (having a hydrophilic moiety introduced therein) having an appropriate specific reactive group therefor (e.g., a carboxy group).

Examples of the amino acid-based polymer include poly(L-lysine), poly(L-arginine), poly(L-ornithine), and poly(L-glutamic acid). The poly(L-lysine) has a structure of -(CH₂)₄-NH₂ at a side chain. The poly(L-arginine) has a structure of -(CH₂)₂-NH-C(=NH)-NH₂ at a side chain. The poly(L-glutamic acid) has a structure of -COOH at a side chain. An amino group: -NH₂ (or a guanidino group: -NH-C(=NH)-NH₂), a carboxy group: -COOH, or the like contained in the side chain of such an amino acid-based polymer serves as the specific reactive group of the high molecular weight polymer and can bind to the phenothiazine-based compound (having a hydrophilic moiety introduced therein) having an appropriate specific reactive group therefor (e.g., a carboxy group or an amino group). The amino acid-based polymer may form a salt, for example, poly(L-arginine hydrochloride) or poly(sodium L-glutamate).

Examples of the polysaccharide-based polymer include cellulose derivatives such as chitosan and carboxymethylcellulose. The chitosan is a polysaccharide obtained by hydrolyzing chitin, and has an amino group at a side chain (in a sugar structure). The carboxymethylcellulose is a cellulose derivative obtained, for example, by alkali-treating cellulose, followed by reaction with monohaloacetic acid, and has a carboxymethyl group at a side chain. An amino group, a carboxy group, or the like contained in the side chain of such a polysaccharide-based polymer serves as the specific reactive group of the high molecular weight polymer and can bind to the phenothiazine-based compound (having a hydrophilic moiety introduced therein) having an appropriate specific reactive group therefor (e.g., a carboxy group or an amino group).

Examples of the protein include enzymes such as glucose dehydrogenase (GDH) and glucose oxidase, each of which can be used as an oxidoreductase in the present invention, and bovine serum albumin (BSA) which is well known and commonly used as a blocking agent. Among amino acid residues contained in such a protein, for example, lysine, arginine, and histidine contain an amino group (or a guanidino group) at a side chain; aspartic acid and glutamic acid contain a carboxy group at a side chain; and cysteine contains a thiol group (sulfanyl group) at a side chain. Thus, the protein containing such an amino acid residue can bind to the phenothiazine-based compound (having a hydrophilic moiety introduced therein) containing an appropriate specific reactive group therefor (e.g., a carboxy group or an amino group).

In a preferred embodiment of the phenothiazine-based compound bound to a polymer according to the present invention, the high molecular weight polymer is a "cationic polymer". The "cationic polymer" is a generic name for polymers having a positive charge as a net of the whole polymer among the high molecular weight polymers as mentioned above. In general, the cationic polymer is a high molecular weight polymer having a positively charged functional group at a side chain, i.e., a high molecular weight polymer synthesized using a monomer having a positively charged functional group at a site that is not involved in backbone formation. Examples of the positively charged functional group include an amino group and a quaternary ammonium cation. A quaternary ammonium cation having chemical stability is preferred. Examples of the cationic polymer include homo copolymers or copolymers comprising a constituent unit derived from 2-aminoethyl methacrylate, (vinylbenzyl)trimethylammonium chloride, or methacryloyl choline chloride, which is disclosed as polymer A, B, or C of Examples, poly(diallyldimethylammonium chloride), poly(allylamine hydrochloride), allylamine hydrochloride-diallylamine hydrochloride copolymers, allylamine-diallyldimethylammonium chloride copolymers, and poly(ethylenimine). Examples of the cationic polymer also include amino acid-based polymers such as poly(L-lysine) and poly(L-arginine), and polysaccharide-based polymers such as chitosan, each of which has an amino group (or a guanidino group) at a side chain.

### <Method for producing phenothiazine-based compound bound to polymer>

The phenothiazine-based compound bound to a polymer according to the present invention can be synthesized by reacting a phenothiazine-based compound (having a hydrophilic moiety introduced therein) having the desired chemical structure with a high molecular weight polymer having the desired chemical structure under appropriate conditions (temperature, time, adjuvant, etc.), particularly, in consideration of the reaction between the specific reactive group of the phenothiazine-based compound or the specific reactive group of a hydrophilic moiety of the phenothiazine-based compound having a hydrophilic moiety introduced therein and the specific reactive group of the high molecular weight polymer. The synthesized phenothiazine-based compound bound to a polymer may be purified, if necessary, by gel filtration chromatography, ultrafiltration, dialysis, or the like to remove low-molecular compounds.

The phenothiazine-based compound bound to a polymer can be produced and used as various types depending on the respective structures of R(L)¹(QP)¹, R(L)²(QP)², and R(L)⁴(QP)⁴ to R(L)⁸(QP)⁸ as well as R⁹ and R¹⁰ in the general formulas (3A-1) and (3B-1). In the present invention, any one type of phenothiazine-based compound bound to a polymer may be used singly or two or more types thereof may be used in combination, for example, as a redox polymer contained in a reagent layer for producing an electrochemical sensor as mentioned later.

### - Reagent layer -

The reagent layer of the present invention is a layer comprising the phenothiazine-based compound having a hydrophilic moiety introduced therein or the phenothiazine-based compound bound to a polymer according to the present invention (hereinafter, collectively referred to as the "phenothiazine-based compound, etc. of the present invention"). This reagent layer is typically disposed on a working electrode in the electrochemical sensor of the present invention. The phenothiazine-based compound (including a compound in the form of the phenothiazine-based compound having a hydrophilic moiety introduced therein or the phenothiazine-based compound bound to a polymer) functions as a redox mediator, and the phenothiazine-based compound bound to a polymer functions as a redox polymer. The details (specific embodiments, preferred embodiments, general formulas, etc.) of the phenothiazine-based compound, etc. of the present invention are as mentioned above in the present specification. The reagent layer may comprise any one type of phenothiazine-based compound, etc. singly or may comprise two or more types of phenothiazine-based compounds, etc. in combination (e.g., as a mixture of two or more types of phenothiazine-based compounds, etc. or in a laminated state of layers containing respective phenothiazine-based compounds, etc.).

The reagent layer may optionally comprise an "oxidoreductase" appropriate for an analyte. An electrochemical sensor typically assumes the form of an electrochemical sensor (biosensor) comprising an oxidoreductase in the reagent layer, though the electrochemical sensor is not limited thereto. The electrochemical sensor may assume the form of an electrochemical sensor other than a biosensor or a sensor (e.g., an optical sensor) other than an electrochemical sensor, comprising no oxidoreductase in the reagent layer. The presence or absence of an oxidoreductase in the reagent layer can be selected depending on the application of an electrochemical sensor or the like, and the type of the oxidoreductase contained can be selected depending on the type of an analyte.

The reagent layer may optionally further comprise an additional component. Examples of the additional component include conductive particles such as carbon particles, and buffer solution components.

The "oxidoreductase" refers to an enzyme that can oxidize or dehydrogenate an analyte targeted by an electrochemical sensor or the like. Examples of the oxidoreductase include oxidase-based enzymes (glucose oxidase, lactate oxidase, pyruvate oxidase, cholesterol oxidase, amino acid oxidase, glutamate oxidase, fructosyl amino acid oxidase, alcohol oxidase, ascorbate oxidase, fructosyl peptide oxidase, bilirubin oxidase, aldehyde oxidase, etc.) and dehydrogenase-based enzymes (glucose dehydrogenase, lactate dehydrogenase, pyruvate dehydrogenase, amino acid dehydrogenase, glutamate dehydrogenase, 3-hydroxybutyrate dehydrogenase, alcohol dehydrogenase, and aldehyde dehydrogenase). Any one of oxidoreductase may be used singly, or two or more thereof may be used, if necessary, in combination.

The oxidoreductase may be cross-linked to the phenothiazine-based compound bound to a polymer according to the present invention. For example, a cross-linking agent (e.g., glutaraldehyde) is reacted with both of a reactive group (e.g., an amino group) carried by the protein oxidoreductase and a reactive group (e.g., an amino group) carried (as a reactive group (for hydrophilic moiety binding) that has not reacted with the phenothiazine-based compound (having a hydrophilic moiety introduced therein), or as a reactive group of different type from that for reaction with the phenothiazine-based compound (having a hydrophilic moiety introduced therein)) by the high molecular weight polymer constituting the phenothiazine-based compound bound to a polymer so that the oxidoreductase can be bound to the phenothiazine-based compound bound to a polymer via the cross-linking agent. The binding of the oxidoreductase allows the phenothiazine-based compound bound to a polymer and the enzyme to become a composite having a larger molecular weight, and can more suppress the leakage of the phenothiazine-based compound (redox mediator) and the enzyme to the outside of a protective film. Oxidoreductases may be cross-linked to each other by using the cross-linking agent. The oxidoreductase, when not cross-linked to the phenothiazine-based compound bound to a polymer, can be contained in a state mixed with the phenothiazine-based compound bound to a polymer in the reagent layer.

The reagent layer can be formed by preparing a reagent solution containing a starting material for forming the reagent layer, and then applying the reagent solution onto a substrate, followed by drying. The reagent solution contains at least the phenothiazine-based compound, etc. of the present invention and can optionally contain an oxidoreductase, a cross-linking agent, and other components (e.g., conductive carbon black). The reagent solution can be prepared by mixing these starting materials, and performing dispersion treatment (e.g., ultrasonication of conductive carbon black or the like which is performed before addition of the oxidoreductase), if necessary. The substrate to which the reagent solution is applied is typically a working electrode of an electrochemical sensor and has, for example, conductive thin films formed by screen printing, sputtering, vapor deposition, ion plating, or the like using a carbon or conductive metal material (gold, silver, platinum, palladium, etc.) on both sides of an insulating substrate. The reagent solution is applied to the surface of this conductive thin film and dried to obtain a working electrode in which the reagent layer is formed on the upper face of the conductive thin film. The reagent layer can be formed with the desired thickness by adjusting the composition of the reagent solution, the area of the applied face, and the amount of the reagent solution applied.

### - Electrochemical sensor -

The electrochemical sensor for detecting or quantifying an analyte according to the present invention has a working electrode, a counter electrode, and the reagent layer of the present invention as mentioned above disposed on the working electrode, and optionally further has a protective film with which at least the reagent layer is coated.

The "protective film" is a membrane-like member for preventing or suppressing the leakage of a substance (the phenothiazine-based compound (having a hydrophilic moiety introduced therein) as a redox mediator, the oxidoreductase, etc.) contained in the reagent layer into an environment (into a living body, into a medium, etc.) outside the protective film. The protective film with which the reagent layer is coated appropriately has pores through which an analyte present in an environment outside the protective film is capable of penetrating the protective film so as to come into contact with the reagent layer. Such a protective film is preferably formed, for example, when the electrochemical sensor is used in sustained measurement, and may not be formed when the electrochemical sensor is used in single measurement.

The working electrode (or a probe having this electrode) on which the reagent layer is formed is inserted to a living body for use or dipped in a medium for use. It is therefore preferred that the protective film which coats the surface thereof have biocompatibility by which proteins or cells are not adsorbed thereto or are difficult to adsorb thereto, and generally be formed from a biocompatible polymer having such properties. Examples of the biocompatible polymer include copolymers of methyl methacrylate and hydroxyethyl methacrylate, copolymers of butyl methacrylate and hydroxyethyl methacrylate, and poly(2-methacryloyloxyethylphosphoryl choline-co-n-butyl methacrylate). The biocompatible polymers described as specific examples of the high molecular weight polymer mentioned above in relation to the phenothiazine-based compound bound to a polymer may be used for forming the protective film.

The protective film can be formed by preparing a solution containing a starting material for forming the protective film (protective film solution), dipping a region where the protective film is to be formed, for example, a region provided with at least the reagent layer on the working electrode, in the solution, pulling up and drying the resultant, and, if necessary, repeating such a process a plurality of times.

The electrochemical sensor of the present invention may optionally further comprise a reference electrode. Specifically, the electrochemical sensor of the present invention may be prepared into a two-electrode system constituted by a working electrode and a counter electrode or may be prepared into a three-electrode system constituted by a working electrode, a counter electrode, and a reference electrode.

In one embodiment of the present invention, the electrochemical sensor can be prepared as an electrochemical sensor of embedded type, for example, a biosensor for CGM (continuous glucose monitoring) for self monitoring of blood glucose which continuously or semi-continuously measures glucose concentrations in blood or interstitial liquid, for example, over several days to several weeks. On the other hand, in one embodiment of the present invention, the electrochemical sensor can also be prepared as an electrochemical sensor of nonembedded type, for example, a biosensor for continuously or semi-continuously measuring concentrations of glucose or the like in a medium.

Hereinafter, embodiments of the electrochemical sensor of the present invention of embedded type will be described with reference to the drawings. However, the drawings and the following description are given for illustrative purposes and do not limit the technical scope of the present invention to the embodiments disclosed thereby. It should be understood that variously changed or modified embodiments are included in the scope of the present invention as long as the objects of the present invention can be attained and as long as the working effects of the present invention are exerted.

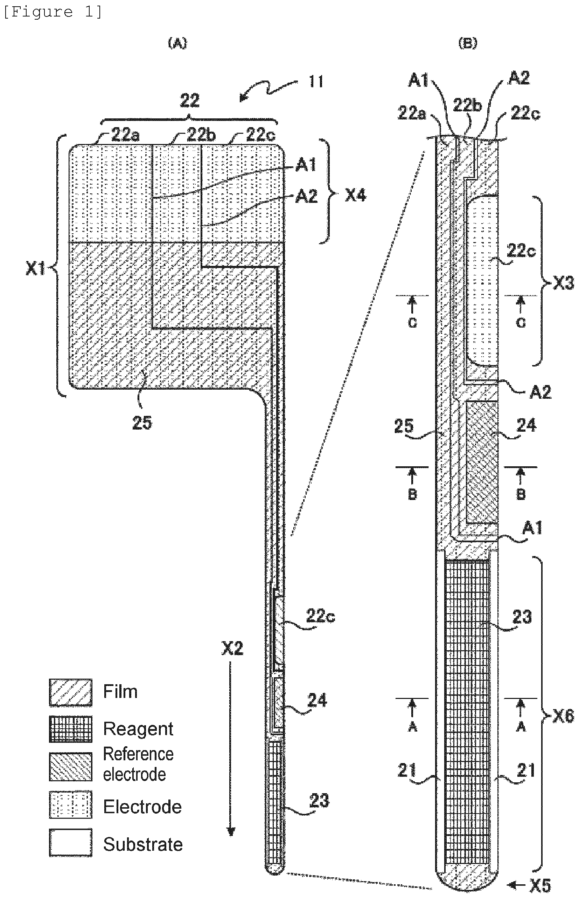

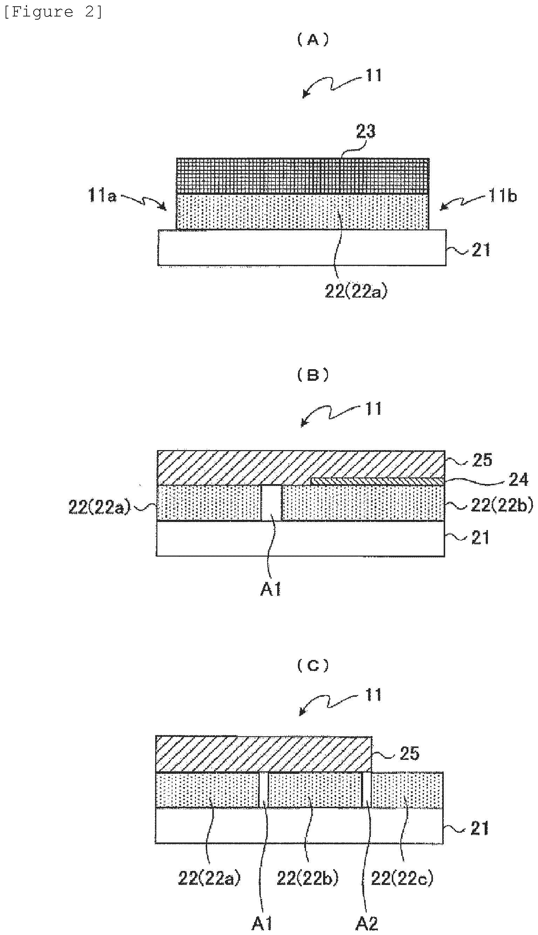

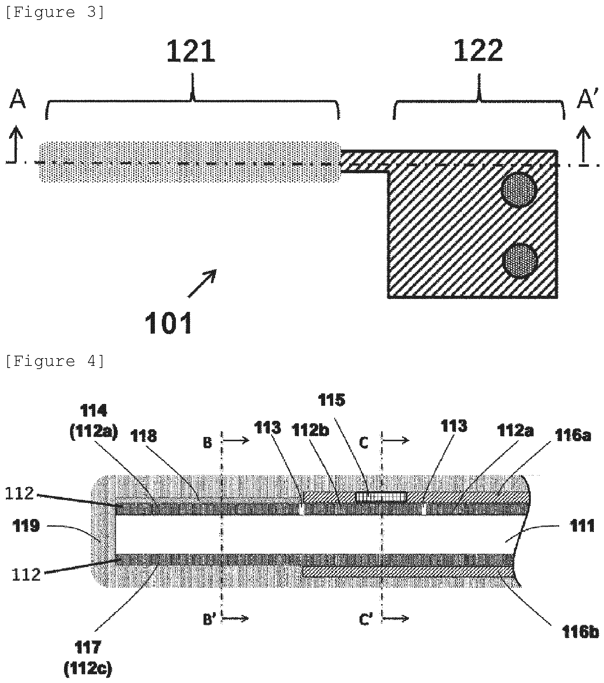

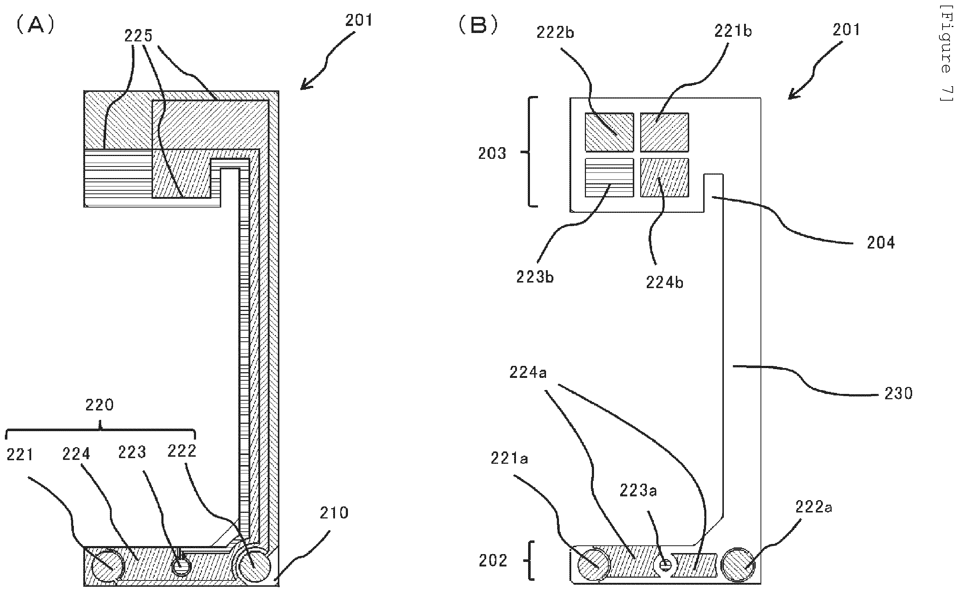

In the description below, a face directed out of the paper in Figures 1(A), 1(B), 3, and 7 or a face directed upward on the paper in Figures 2(A) to 2(C) and 4 to 6 is also referred to as an "upper face"; a face directed into the paper in Figures 1(A) and 1(B), 3, and 7 or a face directed downward on the paper in Figures 2(A) to 2(C) and 4 to 6 is also referred to as a "lower face"; and a face directed to either the left or the right in Figures 1(A), 1(B), 2(A) to 2(C), and 5 to 7 or a face directed either out of or into the paper in Figure 4 is also referred to as a "side face". A dimension in the upward and downward direction of the paper in Figure 1(A), i.e., the direction parallel to the arrow X2 which indicates the direction of insertion of the sensor to a living body, and a dimension in the upward and downward direction of the paper in Figures 3 and 7 are each also referred to as a "length", and a dimension in the left and right direction of the paper in Figure 1(A), i.e., the direction perpendicular to the arrow X2, and a dimension in the upward and downward direction of the paper in Figures 3 and 7 are each also referred to as a "width".

Figure 1 is a plane view of a sensor 11 according to one embodiment of the present invention. Figure 1(A) shows the whole sensor, and Figure 1(B) shows a tip portion (sensing portion) of the sensor 11 shown in Figure 1(A) in an enlarged manner. The sensor 11 is suitable for constructing, for example, a system of a biosensor of embedded type which is attached to a living body for use in self examination of blood glucose levels. In this case, the sensor 11 can be inserted as a protrusion (probe) from the main body (not shown) to a living body. The sensor 11 can also be used for constructing, for example, a system for measuring the concentration of an analyte in a medium.

The region X1 (head) of the sensor 11 shown in is housed in the main body (not shown), and the tip portion (sensing portion) of the sensor 11 protrudes from the main body. Arrow X2 indicates the direction of insertion, for example, when the sensor 11 is inserted to a living body. The dimension of the sensing portion is a length of, for example, 20 to 3 mm, preferably 10 to 3 mm, and a width of, for example, 1 to 50 µm, preferably 500 to 50 µm.

In Figure 1, the sensor 11 has a substrate 21, an electrode 22, a reagent layer 23, a silver/silver chloride layer (also referred to as reference layer) 24, and a film 25. The electrode 22 is uniformly formed on the substrate 21 and comprises a working electrode 22a, a reference electrode 22b, and a counter electrode 22c. The working electrode 22a and the reference electrode 22b are separated physically and electrically through a groove A1, and the reference electrode 22b and the counter electrode 22c are separated physically and electrically through a groove A2. The reagent layer 23 is formed on the working electrode 22a. The silver/silver chloride layer 24 is formed on the reference electrode 22b. The upper face of the sensor 11 except for a portion (in the region X1 of the head, a region X4, a region X3 of the counter electrode 22c, etc.) of the electrode 22 and the reagent layer 23 (by having an opening that exposes them) is coated with the film 25. The exposed region X4 of the electrode 22 is connected with a circuit of the main body 11.

As indicated by a region X5 in Figure 1(B), it is preferred that no reagent layer 23 is formed (over a predetermined distance from the tip) at the tip of the sensor 11. In other words, it is preferred that the reagent layer 23 be distantly formed from the tip of the sensor 11. This is because the detachment (peeling) of the reagent layer 23 from the sensor 11 can thus be suppressed when the sensor 11 is inserted to a living body.

Figure 2(A) is a sectional view taken along the AA line in Figure 1(B). In a portion where the reagent layer 23 is formed in the sensor 11, the substrate 21, the electrode 22 (the working electrode 22a), and the reagent layer 23 are laminated in this order. In portions 11a and 11b, the electrode 22 (the working electrode 22a), the reagent layer 23, and the film 25 are not laminated (are trimmed) to expose the substrate 21.

Figure 2(B) is a sectional view taken along the BB line in Figure 1(B). In a portion where the silver/silver chloride layer 24 is formed on the right side with respect to the groove A1, the substrate 21, the electrode 22 (the reference electrode 22b), the silver/silver chloride layer 24, and the film 25 are laminated. A portion separated physically and electrically from the reference electrode 22b on the left side with respect to the groove A1, the substrate 21, the electrode 22 (the working electrode 22a), and the film 25 are laminated. The side face (right side face in Figure 2(B)) of the silver/silver chloride layer 24 is exposed without being provided with the film 25. In the present embodiment, the upper face of the silver/silver chloride layer 24 is coated with the film 25 or may be exposed without being coated with the film 25.

Figure 2(C) is a sectional view taken along the CC line in Figure 1(B). In a portion on the right side with respect to the groove A2, the substrate 21 and the electrode 22 (the counter electrode 22c) are laminated. The upper face of the counter electrode 22c is exposed without being coated with the film 25. In a portion flanked by the groove A1 and the groove A2, the substrate 21, the electrode 22 (the reference electrode 22b), and the film 25 are laminated. In a portion on the left side of the groove A1, the substrate 21, the electrode 22 (working electrode 22a), and the film 25 are laminated.

The substrate 21 is typically a sheet-shaped synthetic resin. The material of the substrate 21 is not particularly limited as long as the material is a resin material such as a plastic material (synthetic resin) having at least one or more features of flexibility, processability, and heat resistance. Typical examples of such a resin material of the substrate 21 include polyethylene terephthalate (PET). Other examples thereof include general-purpose plastics such as polyethylene, polypropylene, and polyethylene naphthalate. Polyimide is also preferred If high heat resistance is required.

The electrode 22 is a thin film (thin layer) formed on the substrate 21. The material of the electrode 22 is not particularly limited as long as the material is a metal or carbon material having conductivity and stability (e.g., oxidation resistance or salt tolerance). Typical examples of such a material of the electrode 22 include gold. Other examples thereof include platinum, palladium, and carbon. In the case of forming the working electrode 22a (the reagent layer 23) on one of the upper face and the lower face of the sensor 11 and forming the counter electrode 22c on the other face, electrode materials different from each other may be used.

The working electrode 22a is given a potential (potential based on the reference electrode 22b) sufficient for oxidizing a mediator reduced through the reaction of an analyte (e.g., glucose) with an oxidoreductase. Glucose concentrations are measured by monitoring a current flowing between the working electrode 22a and the counter electrode 22c. The reagent layer 23 is formed on the upper face of the working electrode 22a in the tip portion of the sensor 11.

The silver/silver chloride layer 24 is formed, if necessary, on the upper face of the reference electrode 22b in the tip portion of the sensor 11. In the present embodiment, an exemplary three-electrode configuration consisting of the working electrode 22a, the reference electrode 22b, and the counter electrode 23c is shown so as to achieve more accurate measurement. A two-electrode configuration excluding the reference electrode 22b and consisting of the working electrode 22a and the counter electrode 23c is also possible (e.g., such a two-electrode configuration is mainstream for currently commercially available SMBG). The reference electrode can be prepared, as shown in the present embodiment, as a silver/silver chloride electrode provided with the silver/silver chloride (Ag/AgCl) layer 24. In addition, a mercury-containing layer can be formed, as in a hydrogen electrode, a calomel electrode, or the like.

The film 25 is a sheet-shaped insulating member formed (laminated) on a predetermined portion of the electrode 22 (the working electrode 22a, the reference electrode 22b, and the counter electrode 22c) formed on the substrate 21, and the silver/silver chloride layer 24. The thickness of the film 25 is usually 1 µm or larger and 150 µm or smaller, preferably 3 µm or larger and 50 µm or smaller, more preferably 5 µm or larger and 30 µm or smaller. The film 25 has openings in a portion corresponding to the reagent layer 23 located at the tip of the sensor 11, and a portion corresponding to a portion of the counter electrode 22c, and the reagent layer 23 and the counter electrode 22c in the portions are exposed.

The film 25 can be prepared, for example, by affixing a pressure-sensitive adhesive sheet (e.g., acrylic, rubber, or hot-melt type) to the same resin material as that of the substrate 21. The sheet of the same resin material as that of the substrate 21 described above may be a sheet of a resin material different from that of the substrate 21. A single pressure-sensitive adhesive sheet may be used as the film 25. A thermoplastic or photoplastic resist film or a layer formed from resist ink may also be used as the film 25.

A reagent solution for forming the reagent layer 23 can be added dropwise and applied to the surface of the electrode 22 (the working electrode 22a) from the opening located at the corresponding portion of the film 25. The contact angle (α) of the reagent solution with respect to the surface of the film 25 is preferably higher than the contact angle (β) of the reagent solution with respect to the opening of the film 25, i.e., the exposed surface of the working electrode 22a, and the difference therebetween (α - β) is preferably larger, from the viewpoint of the workability of such a process, etc. For example, α is preferably 90° or larger, and β is preferably 50° or smaller. In this context, the "contact angle" is a "static contact angle" measured by the "0/2 method". The material itself forming the film 25 and/or the material itself forming the exposed working electrode 22a may not satisfy the conditions of the contact angle as described above. Even in such a case, the conditions of the contact angle as described above may be satisfied by appropriate surface treatment, for example, at least one of water-repellent treatment of the film 25 and hydrophilic treatment of the working electrode 22a.

Although not shown in Figures 1(A) and 1(B), a portion comprising at least the reagent layer 23 of the sensor 11 may be coated with a protective film. Specifically, a protective film may be formed (laminated) on the upper face of the reagent layer 23 in Figure 2(B). The reagent layer will be mentioned later with reference to Figures 4 to 6 which show the reagent layer.

A method for producing the sensor 11 is not particularly limited, and a method that can produce the sensor 11 having the configurations as mentioned above at predetermined sites can be appropriately selected and used. The sensor 11 can be produced, for example, by performing the following steps (i) to (viii):
(i) forming (laminating) an electrode 22 on the upper face of a substrate 21;
(ii) forming grooves A1 and A2 which physically and electrically separate the electrode 22 into three regions a working electrode 22a, a reference electrode 22b, and a counter electrode 22c;
(iii) forming a silver/silver chloride layer 24 on the upper face of the electrode 22 (the reference electrode 22b) ;
(iv) forming (laminating) a film 25 on the upper faces of the electrode 22 and the silver/silver chloride layer 24;
(v) forming a reagent layer 23 on the upper face of the electrode 22 (the working electrode 22a);
(vi) removing a portion (region X6) of the reagent layer 23 and the electrode 22;
(vii) cutting the sensor 11 out of the substrate 21; and
(viii) forming a protective film.

In the step (i), a method for forming (laminating) the electrode 22 can be appropriately selected and adjusted in consideration of the combination of the material of the electrode 22 and the material of the substrate 21, etc. For example, when the material of the substrate 21 is a synthetic resin such as PET and the material of the electrode 22 is a metal, the electrode 22 made of the metal material can be formed on the surface of the substrate 21 by vapor deposition (including sputtering). Alternatively, printing, plating, spin coating, or the like may be used. When the material of the substrate 21 is a synthetic resin such as PET and the material of the electrode 22 is carbon, the electrode 22 made of carbon can be formed, for example, by printing a carbon paste on the surface of the substrate 21. The substrate 21 in this step does not have to have the shape of the sensor 11 in advance, and the substrate 21 having a larger dimension than that of the sensor 11 can be used such that the sensor 11 can be cut out by the step (vii) performed later.

In the step (ii), for example, laser trimming can be used as an approach of forming the grooves A1 and A2.

In the step (iii), a method for forming the silver/silver chloride layer 24 can be appropriately selected and adjusted in consideration of the combination of the material of the silver/silver chloride layer 24 and the material of the electrode 22, etc. The silver/silver chloride layer 24 can be formed on the upper face of the electrode 22, for example, by printing or applying a silver/silver chloride paste (ink) to the upper face of the electrode 22 made of a metal or carbon material by a method such as screen printing or ink jet, followed by drying. Alternatively, the silver/silver chloride layer 24 may be formed, for example, by printing, applying, or plating silver (Ag) to the upper face of the electrode 22, followed by chlorination treatment of the surface.

In the step (iv), a method for forming (laminating) the film 25 can be appropriately selected and adjusted in consideration of the combination of the material of the film 25 and the materials of the electrode 22 and the silver/silver chloride layer 24, etc. For example, an opening (having a larger dimension than that of at least the reagent layer 23) corresponding to the dimension of the reagent layer 23 is formed in the film 25 made of a laminate of a resin sheet and a pressure-sensitive adhesive sheet or made of a single pressure-sensitive adhesive sheet. The film 25 thus obtained is disposed on the upper face of the electrode 22 such that the opening surrounds a portion where the reagent layer 23 is to be formed in the electrode 22 (working electrode 22a). The film 25 is fixed thereon through the pressure-sensitive adhesive sheet. On the other hand, the film 25 having a predetermined opening can also be formed (laminated) by removing the portion of the predetermined position and dimension as mentioned above using a resist film or resist ink.

No film 25 is formed on the upper face of a portion (the region X3) of the counter electrode 22c in the electrode 22 to expose the counter electrode 22c. Thus, for example, the film may be temporarily formed, also including the upper face of the counter electrode 22c, as described above, and then, an opening in a cutout shape can be formed in the film 25 by cutting or the like.

In the step (v), for example, the film 25 having the predetermined opening and a reagent solution prepared in advance are used, and the reagent solution can be added dropwise to the opening portion of the film 25 formed (laminated) by the step (iv), and thereby applied to at least a portion of the working electrode 22a. Then, the applied reagent solution is dried to form the reagent layer 23 on the upper face of the working electrode 22a.

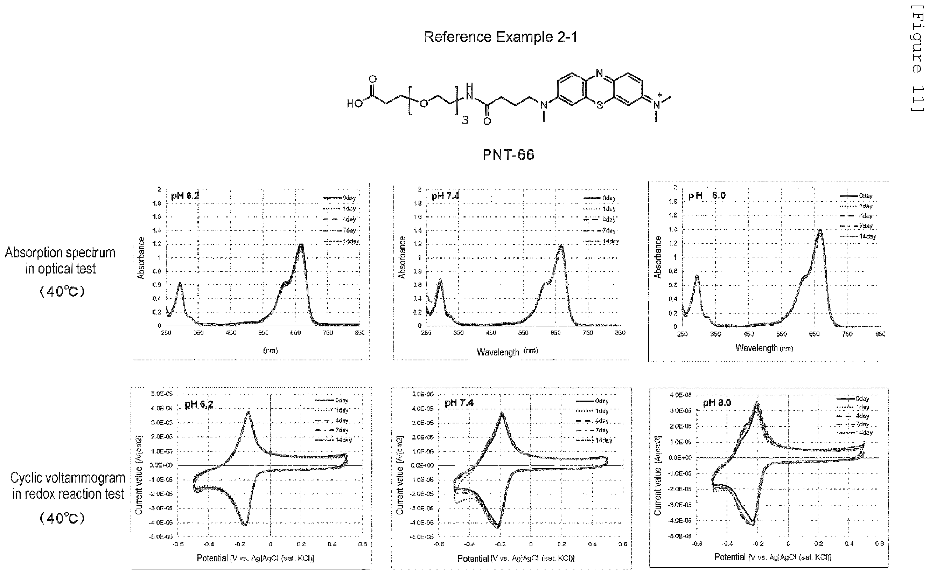

The opening of the film 25 may have, for example, a dimension and a shape that allow a reagent layer having a larger width than that of the sensor 11 (tip portion shown in Figure 11(B)) to be formed. In this case, a reagent layer formed with a larger width than that of the sensor 11 from the applied reagent solution is trimmed such that a predetermined width and shape are attained by the subsequent step (vi).

In the step (vi), the reagent layer 23 and the electrode 22 are removed (trimmed) over a predetermined length in the length direction (e.g., the direction of insertion to a living body) of the sensor 11 at an end in the width direction of the sensor 11 (tip portion). The reagent layer is formed over a certain area by such trimming in the step (vi). Then, a reagent layer having a predetermined area common among sensors is formed by selecting an appropriate portion (preferably a uniform portion). In addition, in the subsequent step (vii), the sensor 11 can be cut out of the substrate 21 along the contour without dividing the formed reagent layer. A method for removing the reagent layer 23 and the electrode 22 can be appropriately selected and adjusted in consideration of the material of the reagent layer 23 (the composition of the reagent solution) and the material of the electrode 22, etc. The reagent layer 23 and the electrode 22 can be removed by, for example, laser trimming.

In the step (vii), a method for cutting the sensor 11 out of the substrate 21 can be appropriately selected and adjusted in consideration of the material of the substrate 21, the shape of the sensor to be cut, etc. For example, when the material of the substrate 21 is a resin, a general cutting technique can be used.

The cutting position includes the portion trimmed by the step (vi) and can be set to, for example, near the center line of a bottom portion of a recess obtained by laser trimming. In other words, cutting is performed at a position distant to a certain degree from the trimmed reagent layer 23 and working electrode 22a.

The step (viii) can be performed with reference to the method for forming a protective film described in relation to the electrochemical sensor of the present invention, and in addition, can be appropriately selected and adjusted in consideration of the combination of the material of the protective film, i.e., the composition of a protective film solution, and the material of the portion to be coated (mainly, the material of the reagent layer 23, i.e., the composition of a reagent solution), the shape or area of the portion to be coated, etc. For example, the working electrode 22a (portion where at least the reagent layer 23 is formed) can be dipped in a protective film solution, pulled up, and dried to coat the portion with a protective film.

Figure 3 is a plane view illustrating a sensor 101 according to another embodiment of the present invention. The sensor 101 is constituted by a sensing portion (tip portion that is inserted to a living body or dipped in a medium) 121, and a terminal portion 122 for electric connection to the internal circuit of the main body (not shown).

Figure 4 shows a sectional view of the sensor 101 taken along the A-A' section line in Figure 3. Conductive thin films 112 are disposed on both sides of an insulating substrate 111. The conductive thin film 112 on one side (front side) of the insulating substrate 111 is separated into a working electrode region 112a and a reference electrode region 112b through electric isolation by forming a groove 113 having a depth that reaches the surface of the insulating substrate 111 by laser lithography.

The upper face of the insulating substrate 111 is coated with an insulating resist film 116a having an opening for forming a reference electrode 115 at a predetermined position of the reference electrode region 112b. The lower face of the insulating substrate 111 is coated with an insulating resist film 116b. On the other hand, neither the upper face nor the lower face is coated with the insulating resist film 116a or 116b from the end of the sensing portion 112 to a given distant location. A region 112a on the front side serves as a working electrode 114, and a region 112c on the back side serves as a counter electrode 117. A reagent layer 118 is formed on the working electrode 114.

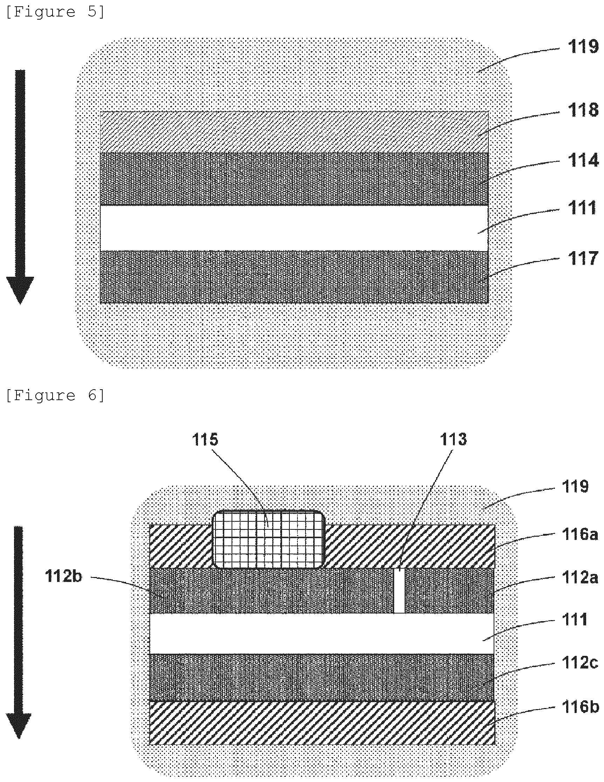

Figure 5 shows a sectional view taken along the B-B' section line in Figure 4. Figure 6 shows a sectional view taken along the C-C' section line in Figure 4. As shown in Figure 5, at the B-B' section line, a protective film 119, a reagent layer 118, a working electrode 114, an insulating substrate 111, a counter electrode 117, and a protective film 119 are formed in order from the upper face toward the lower face (in the direction of the arrow in the drawing). As shown in Figure 6, at the C-C' section line, a protective film 119, a reference electrode 115 (an insulating resist film 116a), a working electrode region 112a, an insulating substrate 111, a counter electrode region 112c, an insulating resist film 116b, and a protective film 119 are formed in order from the upper face toward the lower face (in the direction of the arrow in the drawing). The working electrode region 112a and the counter electrode region 112c shown in Figure 6 have insulating resist films 116a and 116b on the upper face side thereof and thus do not function as a working electrode or a counter electrode.

Figure 7 is a plane view illustrating a sensor 201 according to an alternative embodiment of the present invention. The sensor 201 comprises a sensing portion 202 having a configuration for electrochemically measuring an analyte, a terminal portion 203 having a configuration for electric connection to the internal circuit of the main body (not shown), and a cutout portion 204 provided in the vicinity of the terminal portion. The sensor 201 is an embodiment suitable for constructing a system for analyte measurement in a medium, for example. The sensing portion 202 has a structure dippable in a medium having a small liquid volume. The cutout portion 204 has a structure suitable for placing the sensor 201 in a culture container. Figure 7(A) shows an electrode pattern before formation of a film (insulating resist film) 220. Figure 7(B) shows an electrode pattern after formation of the film 220.

As shown in Figure 7(A), in the sensor 201, an electrode 220 is formed on a substrate 210. The electrode 220 is separated physically and electrically into a first working electrode 221, a second working electrode 222, a reference electrode 223, and a counter electrode 224 through a groove 225. As shown in Figure 7(B), the first working electrode 221 is coated with a film 220 and thereby has an exposed region 221a in the sensing portion 202 and an exposed region 221b in the terminal portion 203. Likewise, the second working electrode 222, the reference electrode 223, and the counter electrode 224 also respectively have exposed regions 222a, 223a, and 224a in the sensing portion 202 and exposed regions 222b, 223b, and 224b in the terminal portion 203. In the sensing portion 202, the exposed region 221a of the first working electrode and the exposed region 222a of the second working electrode are disposed side by side so as to maximize an area, while the exposed region 223a of the reference electrode and the exposed region 224a of the counter electrode are also disposed. Different types of reagent layers (not shown) for measuring different analytes can be formed on the exposed region 221a of the first working electrode and the exposed region 222a of the second working electrode, respectively. The exposed regions 221b, 222b, 223b, and 224b in the respective terminal portions 203 of the first working electrode, the second working electrode, the reference electrode, and the counter electrode can be electrically connected to the main body (not shown) through, for example, an electrode pad (not shown).

### Examples

Hereinafter, embodiments of the present invention will be described further specifically with reference to Examples and Comparative Examples. However, the present invention is not interpreted in a limited manner by the embodiments disclosed in the present Examples, and should be interpreted as encompassing other embodiments that exert working effects of the present invention, as in the embodiments disclosed in the present Examples.

In the following Examples and Comparative Examples, the phenothiazine-based compounds used were 3-(4'-carboxyphenyl)imino-3H-phenothiazine (manufactured by Glanren), thionine (manufactured by FUJIFILM Wako Pure Chemical Corp.), azure A (manufactured by FUJIFILM Wako Pure Chemical Corp.), azure B (manufactured by FUJIFILM Wako Pure Chemical Corp.), azure C (manufactured by MP Biomedicals, Inc. or manufactured by FUJIFILM Wako Pure Chemical Corp.), methylene blue (manufactured by MP Biomedicals, Inc.), and toluidine blue (manufactured by Tokyo Chemical Industry Co., Ltd.). These phenothiazine-based compounds are collectively referred to as "PNT compounds".

Polymer A, polymer B, and polymer C used in the following Examples and Comparative Examples are polymers each obtained by procedures described below.

### [Synthesis of polymer A]

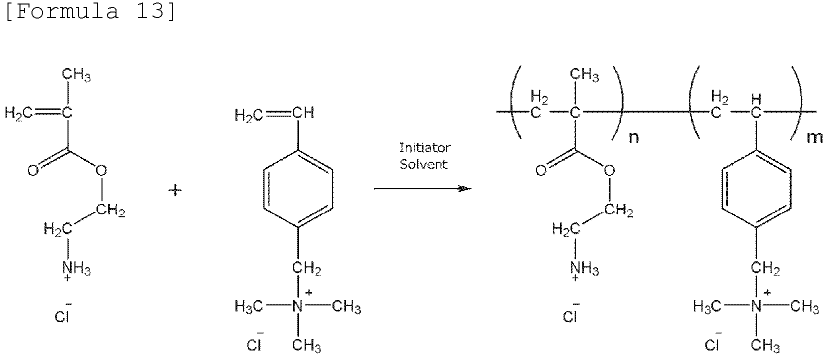

First, 2-aminoethyl methacrylate hydrochloride and (vinylbenzyl)trimethylammonium chloride were provided. Then, a four-neck flask was charged with 7.36 mmol of 2-aminoethyl methacrylate hydrochloride, 7.36 mmol of (vinylbenzyl)trimethylammonium chloride, 0.07 mmol of 2,2'-azobis(2-methylpropionamidine) dihydrochloride (hereinafter, referred to as "V-50"), and 11.11 g of ion exchange water. Subsequently, a stirrer tip, a thermometer, and a pressure reduction/Ar gas line were loaded in the four-neck flask. While stirred at room temperature, the sample in the flask was deaerated by repeating pressure reduction and Ar gas supply 10 times in the four-neck flask using the pressure reduction/Ar gas line. The four-neck flask after deaeration treatment was hermetically sealed, and polymerization reaction was performed at a reaction temperature of 60°C for 24 hours (the reaction time was appropriately adjusted according to need). The progression and completion of polymerization were confirmed by measuring the consumption of the monomers by 1H-NMR. After the completion of reaction, the reaction solution was placed in a dialysis membrane (MWCO: 3500) and dialyzed for 3 days. During this operation, the solution was replaced twice a day. After the completion of dialysis, the obtained reaction product was freeze-dried to obtain polymer A. The polymer A corresponds to a polymer with n:m = 48.5:51.5 as a result of calculating a molar ratio from the results of 1H-NMR.

### [Synthesis of polymer B]

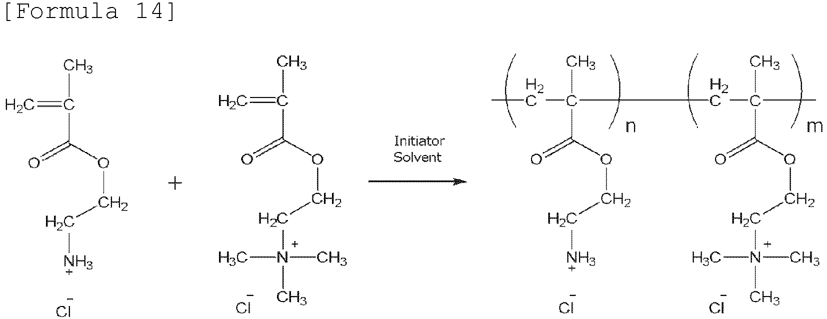

First, 2-aminoethyl methacrylate hydrochloride and methacryloyl choline chloride were provided. Then, a four-neck flask was charged with 1.14 mmol of 2-aminoethyl methacrylate hydrochloride, 10.22 mmol of methacryloyl choline chloride, 0.06 mmol of V-50, and 8.71 g of ion exchange water. Thereafter, the same treatment as in the polymer A was performed to obtain polymer B. The polymer B corresponds to a polymer with n:m = 13.3:86.7 as a result of calculating a molar ratio from the results of 1H-NMR.

### [Synthesis of polymer C]

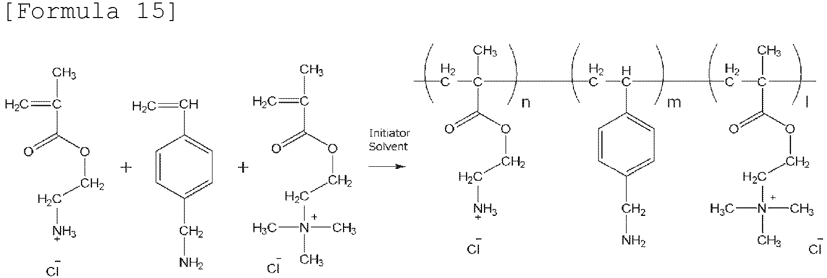

First, 2-aminoethyl methacrylate hydrochloride, (4-vinylphenyl)methanamine, and methacryloyl choline chloride were provided. Then, a four-neck flask was charged with 0.97 mmol of 2-aminoethyl methacrylate hydrochloride, 0.97 mmol of (4-vinylphenyl)methanamine, 11.97 mmol of methacryloyl choline chloride, 0.08 mmol of V-50, and 10.49 g of ethanol. Thereafter, the same treatment as in the polymer A was performed to obtain polymer C. The polymer C corresponds to a polymer with n:m:l = 21.2:8.1:70.7 as a result of calculating a molar ratio from the results of 1H-NMR. The polymer had number-average molecular weight Mn of 37774 and weight-average molecular weight Mw of 214367 as a result of measurement by gel permeation chromatography (GPC).

### [Reference Examples 1-1 to 1-5 and Reference Comparative Examples 1-1 and 1-2]

An optical test and a redox reaction test were conducted in accordance with procedures described below using 3-(4'-carboxyphenyl)imino-3H-phenothiazine (Reference Comparative Example 1-1), thionine (Reference Comparative Example 1-2), azure A (Reference Example 1-1), azure B (Reference Example 1-2), azure C (Reference Example 1-3), methylene blue (Reference Example 1-4), and toluidine blue (Reference Example 1-5) as PNT compounds.

### <Optical test>

Each PNT compound was adjusted to a suitable concentration in the range of 0.01 to 0.2 mM with a 100 mM sodium phosphate buffer (pH 7.4) to prepare an aqueous solution. An absorption spectrum was measured using VIOLAMO Disposable Cell Semimicro Type Ultraviolet Rays Transmission Type and Agilent 8453 UV-visible Spectroscopy System. The aqueous solution of each PNT compound after measurement was preserved in an environment of 40°C and then taken out as needed for measurement to confirm time-dependent change up to day 14 or 15.

### <Redox reaction test>

Redox reaction was measured by cyclic voltammetry. The measurement was performed in a potentiostat using each aqueous PNT solution adjusted to 0.1 mM with a 100 mM sodium phosphate buffer (pH 7.4) and using a glassy carbon electrode as a working electrode, a platinum wire in a counter electrode, and a Ag/AgCl electrode (saturated KCl) as a reference electrode. A scan rate was set to 0.1 V/Sec. Various electrodes and a potentiostat manufactured by BAS Inc. were used as those generally used in electrochemistry. The aqueous solution of each PNT compound after measurement was preserved in an environment of 40°C and then taken out as needed for measurement to confirm time-dependent change up to a predetermined number of days.

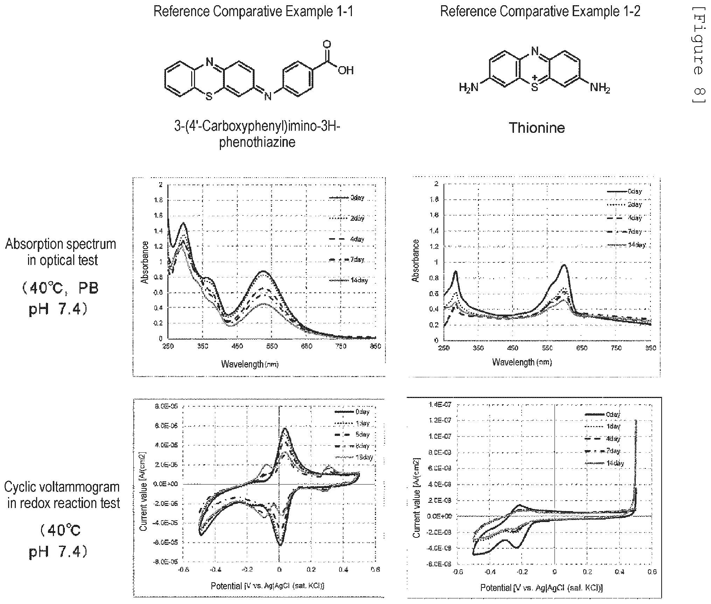

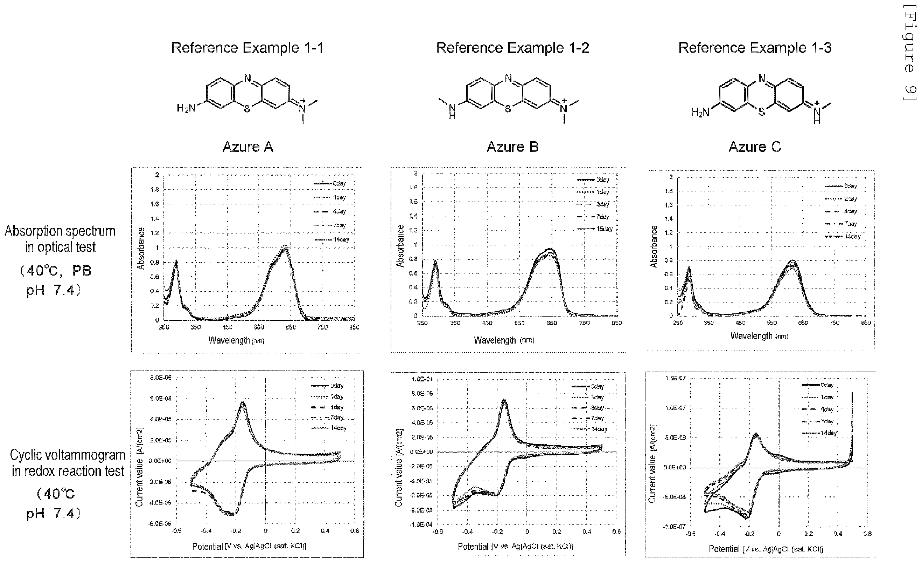

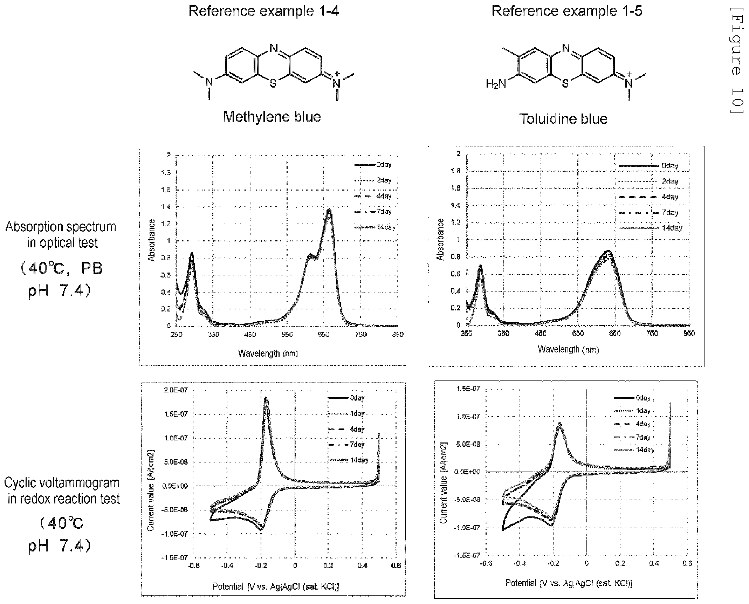

The results of the tests are shown in Figures 8, 9, and 10. From the absorption spectrum and the cyclic voltammogram measured over time, PNT compounds having an iminium cation at the 3-position of a phenothiazine skeleton (Reference Examples 1-1 to 1-5) were found to have high preservation stability as compared with a PNT compound having an imino group at this position (Reference Comparative Example 1-1) and a PNT compound having an amino group at this position (Reference Comparative Example 1-2).

### [Example 2-1] PNT compound having hydrophilic moiety (-N(CH₃)(CH₂)₃-CO-NH-PEG3-(CH₂)₂-COOH) derived from compound for hydrophilic moiety introduction, bound to specific binding substituent (PNT-66)

### (1) Synthesis of sulfonic acid fragment

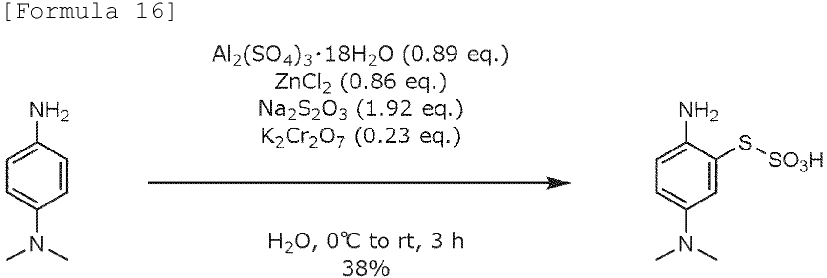

A sulfonic acid fragment was synthesized through the reaction described above.

### (2) Synthesis of sulfonic acid fragment

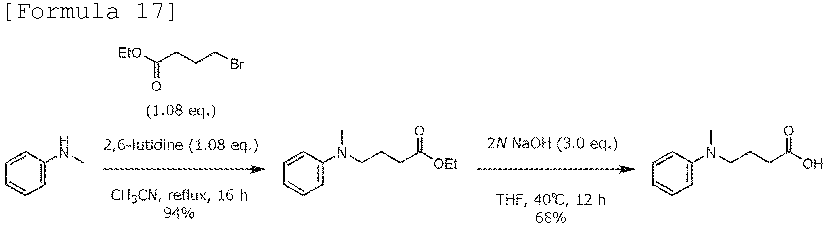

A carboxylic acid fragment was synthesized through the reaction described above.

### (3) Synthesis of PNT-34

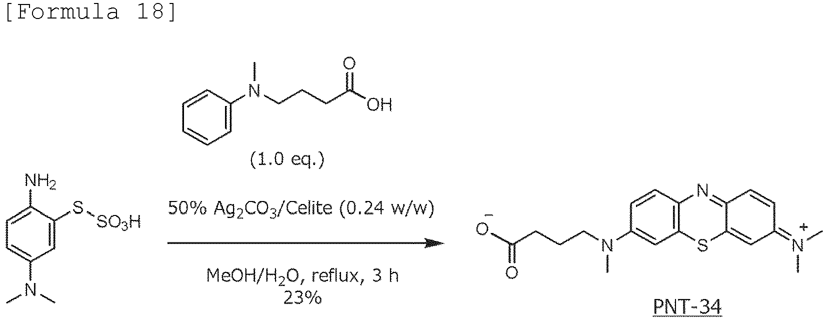

The sulfonic acid fragment and the carboxylic acid fragment synthesized as described above were suspended in MeOH/H₂O, and 50% Ag₂CO₃/celite was added thereto in divided portions over 15 minutes at an internal temperature around 47°C. After addition, the mixture was stirred at an internal temperature around 68°C for 2.5 hours. The reaction mixture was allowed to cool to room temperature and filtered through celite, and the filtrate was concentrated. The residue was purified a plurality of times by silica gel column chromatography to obtain PNT-34.

### (4) Synthesis of condensate (PNT-65)

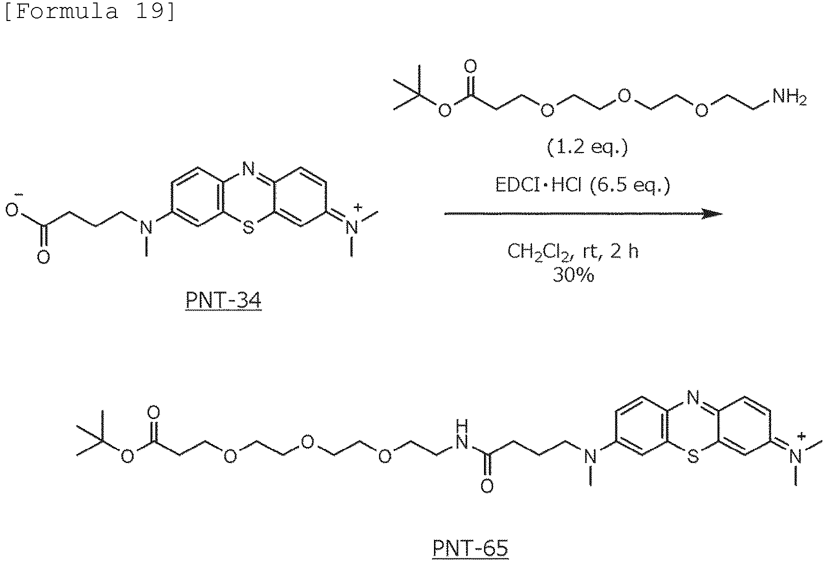

PNT-34 was dissolved in dichloromethane to prepare a solution (1). Amino-PEG3-t-butyl ester and 1-ethyl-3-(3-dimethylaminopropyl)carbodiimide hydrochloride (EDCI·HCl) were suspended in dichloromethane to prepare a suspension (2). The suspension (2) was added to the solution (1), and the mixture was stirred at room temperature for 2 hours. The reaction solution was purified twice by silica gel column chromatography to obtain a condensate (PNT-65).

### (5) Synthesis of deprotected form (PNT-66)

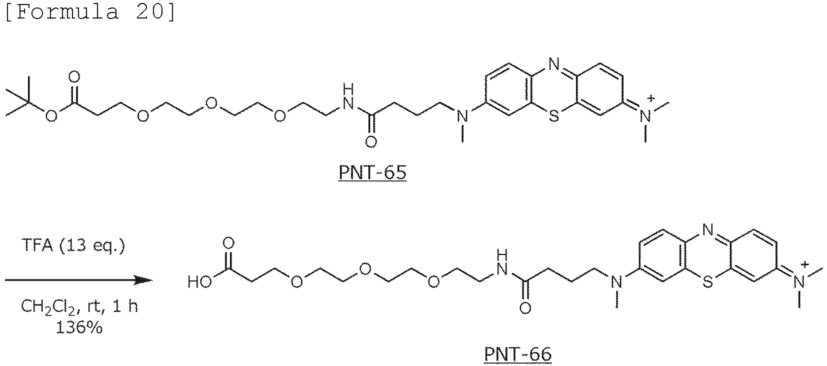

The condensate (PNT-65) was dissolved in dichloromethane. To the solution, TFA was added, and the mixture was then stirred at room temperature for 1 hour. The reaction solution was concentrated and then subjected to azeotropy with toluene several times to obtain a deprotected form (PNT-66).

### [Example 2-2] PNT compound having hydrophilic moiety (-N(CH₃)(CH₂)₃-CO-NH-PEG12-(CH₂)₂-COOH) derived from compound for hydrophilic moiety introduction, bound to specific binding substituent (PNT-69)

### (1) Synthesis of condensate (PNT-68)

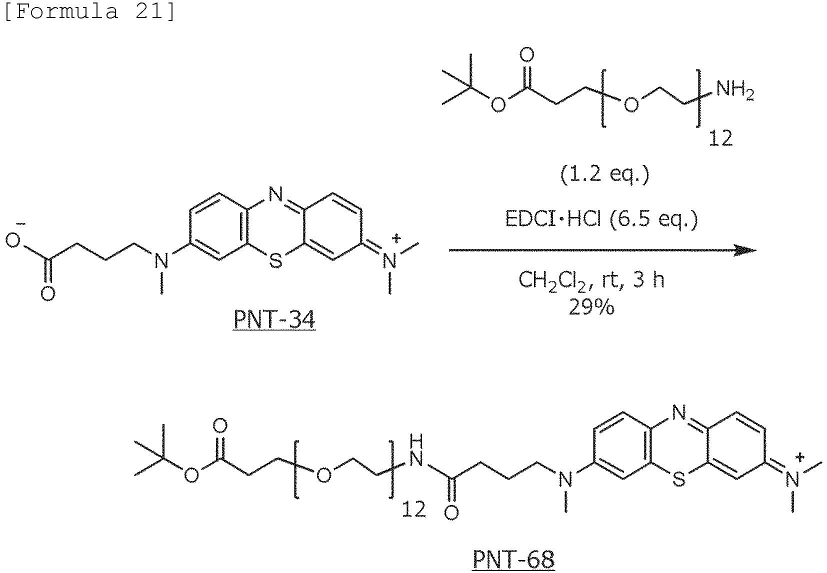

In an Ar atmosphere, PNT-34 (see Example 2-1) was dissolved in dichloromethane. To the solution, amino-PEG12-t-butyl ester and EDCI·HCl were added, and the mixture was then stirred at room temperature for 3 hours. The reaction solution was concentrated and then purified by silica gel column chromatography three times to obtain a condensate (PNT-68).

### (2) Synthesis of deprotected form (PNT-69)

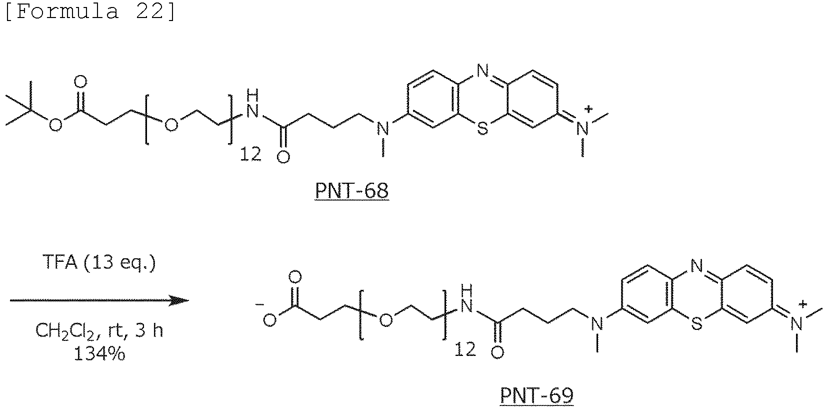

The condensate (PNT-68) obtained in (1) was dissolved in dichloromethane. To the solution, TFA was added, and the mixture was then stirred at room temperature for 3 hours. The reaction solution was concentrated and then subjected to azeotropy with toluene several times to obtain a deprotected form (PNT-69).

### [Example 2-3] PNT compound having hydrophilic moiety (-N(CH₃)CH₂-CO-NH-PEG12-CH₂-COOH) derived from compound for hydrophilic moiety introduction, bound to specific binding substituent (PNT-63)

### (1) Step 1: Synthesis of phenothiazine-based compound through alkylation reaction

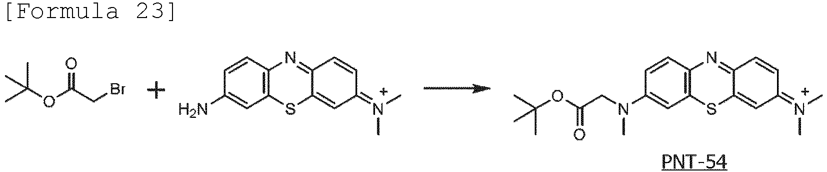

100 mg (343.57 µmol) of azure A was dissolved (suspended) in 2 mL of DMF. To the solution, 355 mg (1,710.9 µmol) of t-butyl bromoacetate and subsequently 226 mg (1,635 µmol) of potassium carbonate (K₂CO₃) were added, and the mixture was stirred at room temperature for 3 hours. 244 mg (1,718 µmol) of methyl iodide was added thereto, and the mixture was stirred at room temperature for 2 hours. Insoluble matter was filtered off, and the filtrate was then purified with a silica gel column (methanol/chloroform, stepwise) followed by an ODS column (0.1% TFA/methanol, linear gradient) to obtain 40 mg of PNT-54.

### (2) Step 2: Deprotection of substituent for hydrophilic moiety introduction and introduction of hydrophilic moiety through amidation reaction

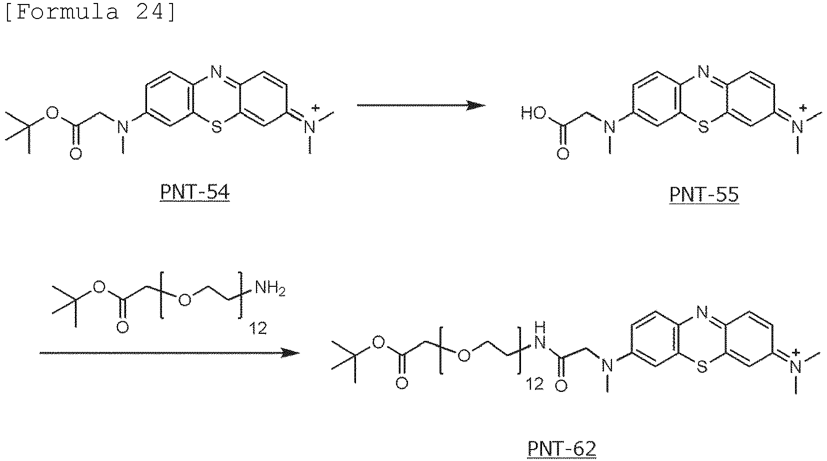

1 mL of TFA was added to 53 mg of PNT-54, and the mixture was stirred at room temperature for 1 hour. TFA was removed under reduced pressure, and a DMF solution containing 40.5 mg (96.4 µmol) of NH₂-PEG13-COOtBu and 123 mg (642.6 µmol) of WSC dissolved therein was then added thereto at once. The mixture was stirred at room temperature for 1 hour and then concentrated under reduced pressure to remove DMF. The residue was purified with an ODS column followed by a HPLC column (0.1% TFA/water, acetonitrile gradient) to obtain 17.7 mg of PNT-62.

### (3) Step 3: Deprotection of hydrophilic moiety

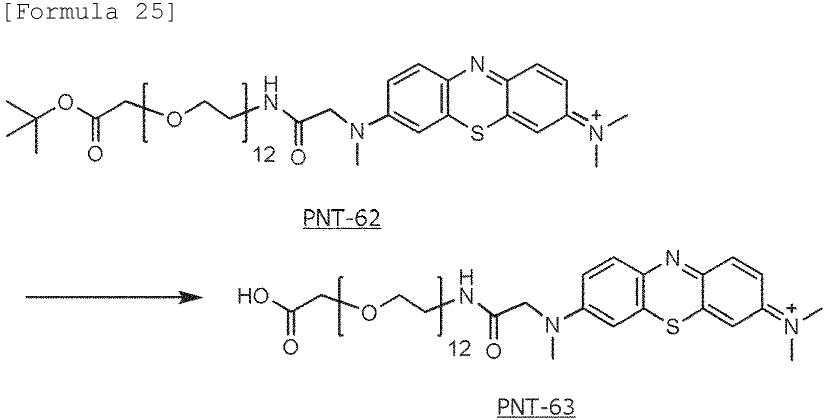

1 mL of TFA was added to 21 mg of PNT-62, and the mixture was stirred at room temperature for 1 hour. TFA was removed under reduced pressure, and the residue was directly dried under reduced pressure to obtain 20 mg of the target compound PNT-63.

### <Optical test and redox reaction test>

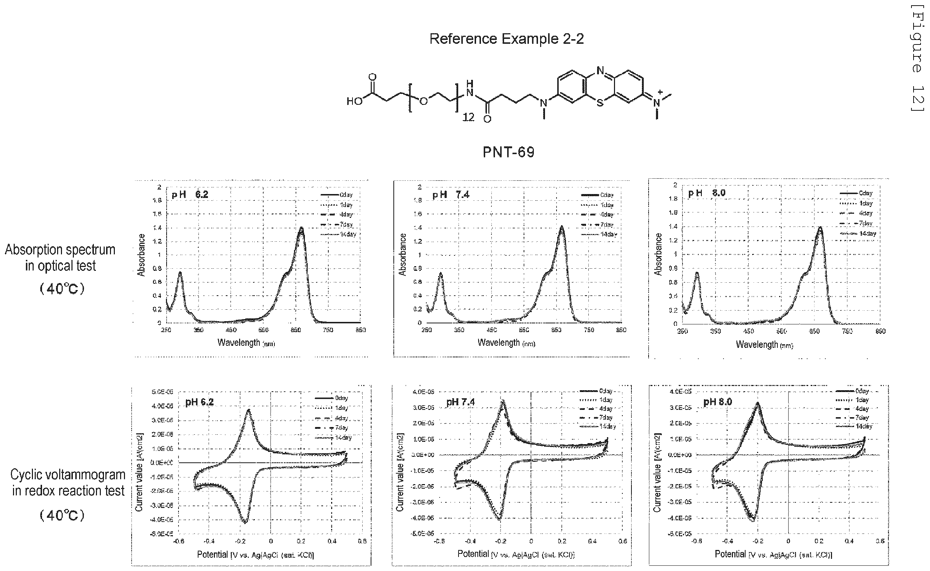

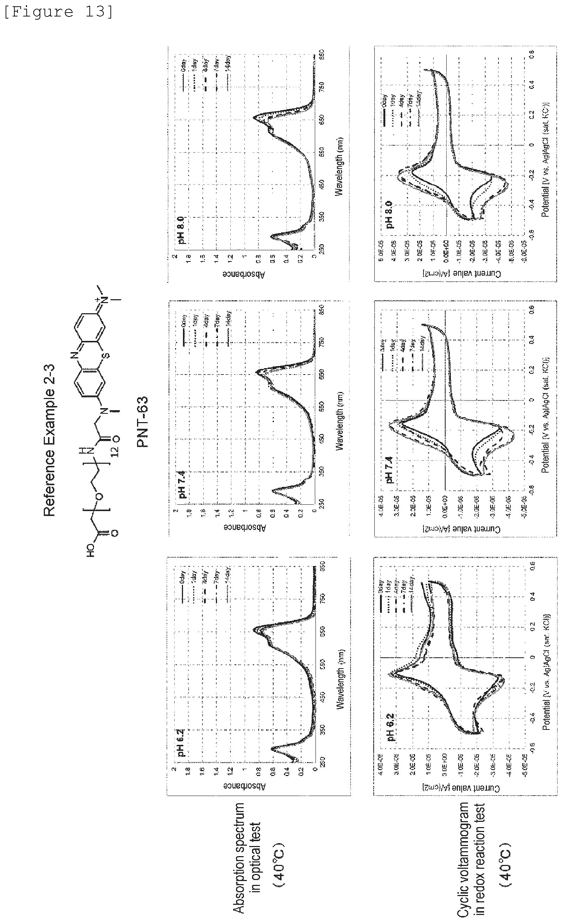

An optical test and a redox reaction test were conducted in the same manner as the procedures in the aforementioned Reference Examples and Reference Comparative Examples except that each of the deprotected forms of Examples 2-1 to 2-3 was tested using a sodium phosphate buffer of pH 7.4 as well as sodium phosphate buffers of pH 6.2 and pH 8.0. The results are shown in Figures 11, 12, and 13. From the absorption spectrum and the cyclic voltammogram measured over time, PNT compounds having an iminium cation at the 3-position of a phenothiazine skeleton and also having a hydrophilic moiety (PEG chain) introduced at another site (6-position) of the phenothiazine skeleton were also found to have high preservation stability.

### [Example 3-1] Preparation of PNT-66 bound to poly(L-lysine)

### (1) Synthesis of PNT-66NHS form (PNT-67)

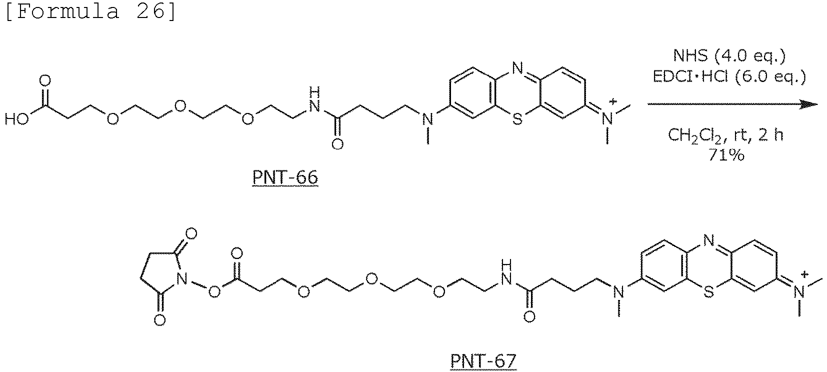

A deprotected form (PNT-66) was dissolved in dichloromethane. To the solution, N-hydroxysuccinimide (NHS) and EDCI·HCl were added, and the mixture was then stirred at room temperature for 2 hours. The reaction solution was purified twice by silica gel column chromatography to obtain a PNT-66NHS form having an activated (NHS esterified) carboxy group at the end of a PEG chain (PEG3) (PNT-67).

### (2) Binding to poly(L-lysine)

The PNT-66NHS form (PNT-67) obtained in (1) was dissolved at 15 mg/mL in MiliQ water (liquid (1)). Poly(L-lysine) hydrochloride (ALAMANDA POLYMERS PLKC800) was dissolved as a high molecular weight polymer at 10 mg/mL in MiliQ water (liquid (2)). Next, WSC (DOJINDO W001) was dissolved at 20 mg/mL in MiliQ water (liquid (3)). 43.7 µL of the liquid (1), 51.3 µL of the liquid (2), 479.3 µL of the liquid (3), and 96 µL of a 250 mM 2-morpholinoethanesulfonic acid (MES) buffer solution (pH 6.0) prepared separately were mixed and adjusted to a total volume of 1200 µL with MiliQ water. Then, the mixture was reacted with stirring at room temperature for approximately 20 hours. Then, ultrafiltration was performed several times using a centrifugal ultrafiltration filter (Amicon Ultra-4 50k; Merck Millipore) to recover a liquid free from small molecules.

### [Example 3-2] Preparation of PNT-69 bound to polymer A (1) Synthesis of PNT-69NHS form (PNT-70)

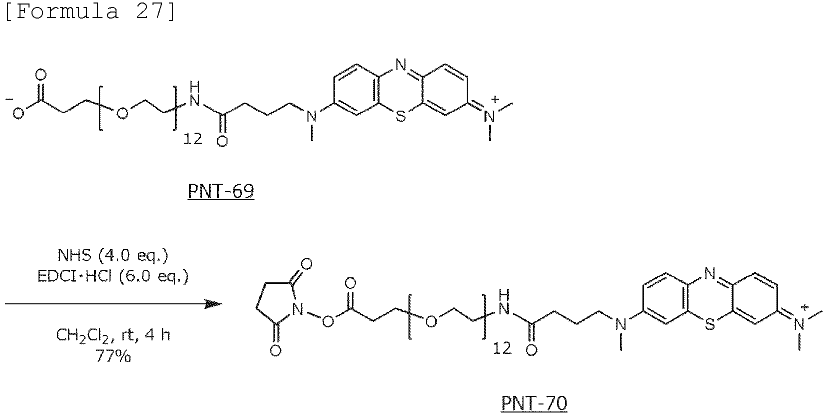

A deprotected form (PNT-69) was dissolved in dichloromethane. To the solution, N-hydroxysuccinimide (NHS) and EDCI·HCl were added, and the mixture was then stirred at room temperature for 4 hours. The reaction solution was purified by silica gel column chromatography to obtain a PNT-69NHS form having an activated (NHS esterified) carboxy group at the end of a PEG chain (PEG12) (PNT-70).

### (2) Binding to polymer A

A PNT-69NHS form (PNT-70) was dissolved at 21.05 mg/mL in MiliQ water (liquid (1)). Polymer A was dissolved as a high molecular weight polymer at 15 mg/mL in MiliQ water (liquid (2)). Next, WSC (DOJINDO W001) was dissolved at 20 mg/mL in MiliQ water (liquid (3)). 50 µL of the liquid (1), 104.1 µL of the liquid (2), 479.3 µL of the liquid (3), and 96 µL of a 250 mM 2-morpholinoethanesulfonic acid (MES) buffer solution (pH 6.0) prepared separately were mixed and adjusted to a total volume of 1200 µL with MiliQ water. Then, the mixture was reacted with stirring at room temperature for approximately 20 hours. Then, ultrafiltration was performed several times using a centrifugal ultrafiltration filter (Amicon Ultra-4 50k; Merck Millipore) to recover a liquid free from small molecules.

### [Example 3-3] Preparation of PNT-69 bound to polymer B

A PNT-69NHS form (PNT-70) was dissolved at 21.05 mg/mL in MiliQ water (liquid (1)). Polymer B was dissolved as a high molecular weight polymer at 15 mg/mL in MiliQ water (liquid (2)). Next, WSC (DOJINDO W001) was dissolved at 20 mg/mL in MiliQ water (liquid (3)). 50 µL of the liquid (1), 405.2 µL of the liquid (2), 479.3 µL of the liquid (3), and 96 µL of a 250 mM 2-morpholinoethanesulfonic acid (MES) buffer solution (pH 6.0) prepared separately were mixed and adjusted to a total volume of 1200 µL with MiliQ water. Then, the mixture was reacted with stirring at room temperature for approximately 20 hours. Then, ultrafiltration was performed several times using a centrifugal ultrafiltration filter (Amicon Ultra-4 50k; Merck Millipore) to recover a liquid free from small molecules.

### [Example 3-4] Preparation of PNT-69 bound to polymer C

A PNT-69NHS form (PNT-70) was dissolved at 21.05 mg/mL in MiliQ water (liquid (1)). Polymer C was dissolved as a high molecular weight polymer at 15 mg/mL in MiliQ water (liquid (2)). Next, WSC (DOJINDO W001) was dissolved at 20 mg/mL in MiliQ water (liquid (3)). 40 µL of the liquid (1), 140.3 µL of the liquid (2), 383.4 µL of the liquid (3), and 96 µL of a 250 mM 2-morpholinoethanesulfonic acid (MES) buffer solution (pH 6.0) prepared separately were mixed and adjusted to a total volume of 1200 µL with MiliQ water. Then, the mixture was reacted with stirring at room temperature for approximately 20 hours. Then, ultrafiltration was performed several times using a centrifugal ultrafiltration filter (Amicon Ultra-4 50k; Merck Millipore) to recover a liquid free from small molecules.

### <Optical test>

As for PNT-66 or PNT-69 bound to a high molecular weight polymer (polymer-bound PNT) in each of Examples 3-1 to 3-4, a recovered solution of the polymer-bound PNT was adjusted to a predetermined concentration with phosphate-buffered saline (PBS) (Takara Bio PBS Tablets T9181) and added at 150 µL to a microplate. An absorption spectrum was measured using a plate reader. The solution after measurement was preserved at 37°C and taken out at days 7 and 14 for measurement again. Here, the microplate used was UV-Star(R) 96 Well F-Boden manufactured by Greiner Bio-One. The plate reader used was infinite(R) M200 Pro manufactured by Tecan Group Ltd.

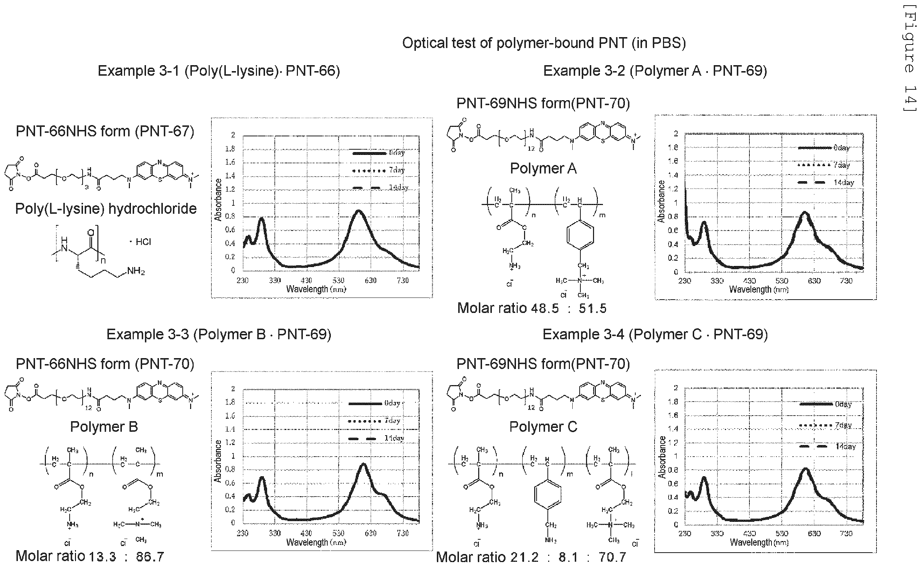

The results are shown in Figure 14. From the measurement results, no marked change in spectrum shape even on day 14 after the start of preservation at 37°C showed that PNT after binding to various polymers has high preservation stability.

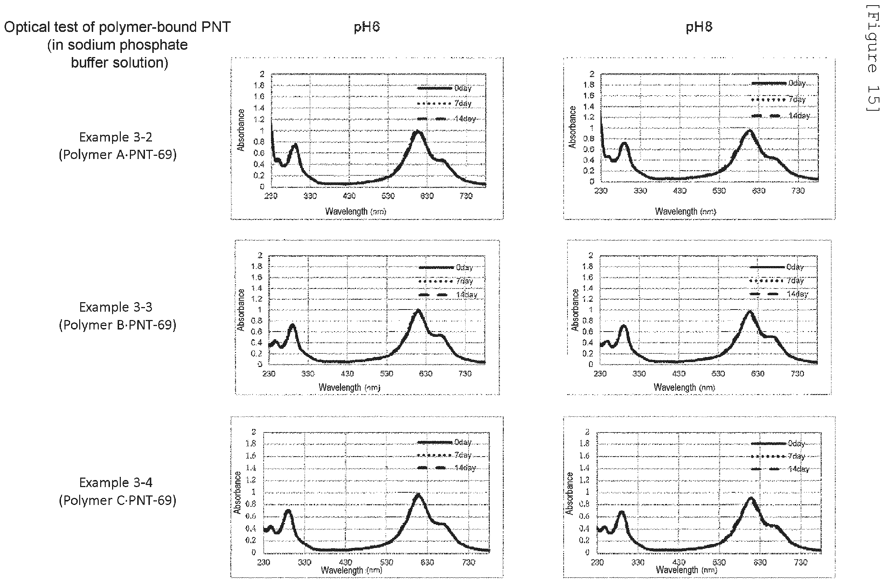

As for the polymer-bound PNT (PNT-69 bound to high molecular weight polymer A, B, or C) prepared in each of Examples 3-2 to 3-4, an absorption spectrum was measured in the same manner as above except that a sodium phosphate buffer solution of pH 6 or pH 8 (prepared with disodium hydrogen phosphate and sodium dihydrogen phosphate) was used instead of phosphate-buffered saline (PBS). The results are shown in Figure 15. These three types of polymer-bound PNT also had no marked change in spectrum shape even on day 14 after the start of preservation at 37°C in a sodium phosphate buffer solution of pH 6 or pH 8, as in PBS, and was thus shown to have high preservation stability.

### <Electrochemical response test>

### (1) Preparation of reagent of polymer-bound PNT

The respective reagents of the polymer-bound PNT prepared in each of Examples 3-2 to 3-4 and a carbon dispersion were mixed so as to attain the composition shown in Table 2. The mixture was reacted for approximately 1 hour to prepare a reagent. The carbon dispersion was prepared by mixing Ketjen Black EC300J (Lion Specialty Chemicals Co., Ltd.) at a carbon concentration of 16 mg/mL with a 5 mg/mL hydroxypropylcellulose (NISSO HPCL) solution, and treating the mixture in an ultrasonic homogenizer for 3 minutes or longer. After preparation of the dispersion and before use thereof in reagent preparation, the dispersion was treated in an ultrasonic bath for approximately 10 minutes. The concentration of each polymer-bound PNT was adjusted by diluting each solution 25-fold, adding the dilution at 100 µL to a microplate, measuring an absorption spectrum using a plate reader to confirm the concentration of the stock solution, and adjusting the concentration on the basis of the obtained value.

**[Table 2]**

| Reagent name | Final concentration | |
|---|---|---|
| Carbon dispersion | 5 | mg/mL |
| Each polymer-bound PNT | 2 | Absorbance at 608 nm |

### (2) Preparation of sensor

0.4 µL of the reagent prepared in (1) was applied onto a gold electrode prepared by sputtering on an insulating substrate, and dried for approximately 15 minutes. Again, 0.4 µL of the reagent was applied thereto and then dried overnight to obtain an electrode having a reagent layer containing the polymer-bound PNT (polymer-bound PNT-coated electrode).

### (3) Evaluation

Cyclic voltammetry was performed in PBS using a potentiostat manufactured by BAS Inc. in a three-electrode system using the polymer-bound PNT-coated electrode prepared in (2) as a working electrode, a gold electrode as a counter electrode, and a Ag/AgCl electrode (saturated KCl) (manufactured by BAS Inc.) as a reference electrode. A scan rate was set to 10 mV/s, and redox peak current values were also measured at each scan rate.

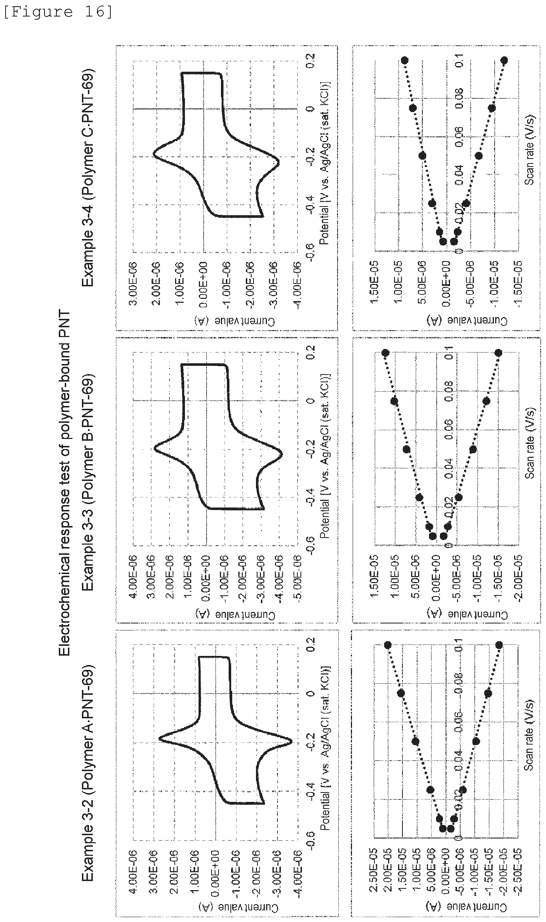

The results are shown in Figure 16. From the obtained cyclic voltammogram, redox currents were observed, demonstrating that PNT also has functions as a mediator after polymer binding. The oxidation peak current value (Ipa) and the reduction peak current value (Ipc) were proportional to the scan rate, suggesting that PNT after polymer binding is adsorbed onto an electrode.

### <Electrochemical response and durability test>

### (1) Preparation of reagent of polymer-bound PNT

A reagent of polymer-bound PNT was prepared in the same manner as in the "electrochemical response test" mentioned above.

### (2) Preparation of sensor

A sensor was prepared in the same manner as in the "electrochemical response test" mentioned above.

### (3) Preparation of polymer solution for protective film

Reagents shown below were mixed at the following final concentrations to prepare a polymer solution for a protective film.
Poly(ter.butyl methacrylate-b-4-vinylpyridine) (manufactured by Polymer Source, Inc.; hereinafter, referred to as "tBuMA4VP"), final concentration: 7.11% (wt/v)
Tripropylene glycol methyl ether methacrylate-styrene-4-vinylpyridine random copolymer (manufactured by NARD Institute, Ltd.; hereinafter, referred to as "TGMAS4VP"), final concentration: 0.89% (wt/v)
Poly(ethylene glycol) diglycidyl ether (manufactured by Sigma-Aldrich Co., LLC; hereinafter, referred to as "PEGDGE"), final concentration: 0.98% (wt/v)
HEPES buffer solution (pH 8.0), final concentration: 5 mM

tBuMA4VP, TGMAS4VP, and PEGDGE were dissolved in ethanol for use. The number-average molecular weight Mn of tBuMA4VP was 87,000 for poly(ter.butyl methacrylate) and 74,000 for poly(4-vinylpyridine). Mw/Mn of tBuMA4VP was 1.16. TGMAS4VP had tripropylene glycol methyl ether methacrylate:styrene:4-vinylpyridine = 6.6:20.3:73.0, number-average molecular weight Mn of 60,704, weight-average molecular weight Mw of 120,095, and Mw/Mn of 1.98. The number-average molecular weight Mn of PEGDGE was up to 1,000. The HEPES buffer solution was prepared using 2-[4-(2-hydroxyethyl)-1-piperazinyl]ethanesulfonic acid (manufactured by Dojindo Laboratories).

### (4) Coating film of polymer solution for protective film

The electrode (polymer-bound PNT-coated electrode) prepared in (2) was dipped in a polymer solution for a protective film, pulled up, and dried. This operation was repeated a plurality of times to form a protective film on each electrode.

### (5) Evaluation

Cyclic voltammetry was performed in PBS or RPMI medium using a potentiostat manufactured by BAS Inc. in a three-electrode system using the electrode prepared in (4) as a working electrode, a gold electrode as a counter electrode, and a Ag/AgCl electrode (saturated KCl) (manufactured by BAS Inc.) as a reference electrode. A scan rate was set to 10 mV/s. In the measurement, a final cyclic voltammogram was obtained by circling a plurality of times in cyclic voltammetry. The electrode after measurement was preserved in each solution (the same as the measurement solution) of 37°C. The same measurement as above was performed on days 1, 4, 7, and 19 after the start of preservation. The RPMI medium used was a liquid obtained by adding MES (2-morpholinoethanesulfonic acid monohydrate) (manufactured by Dojindo Laboratories) and MOPS (3-morpholinopropanesulfonic acid) (manufactured by Dojindo Laboratories) at their final concentrations of 25 mM as buffer solution components to a solution prepared with RPMI-1640 Medium (R1383 manufactured by Sigma-Aldrich Co., LLC), and adjusting the pH to 7.4.

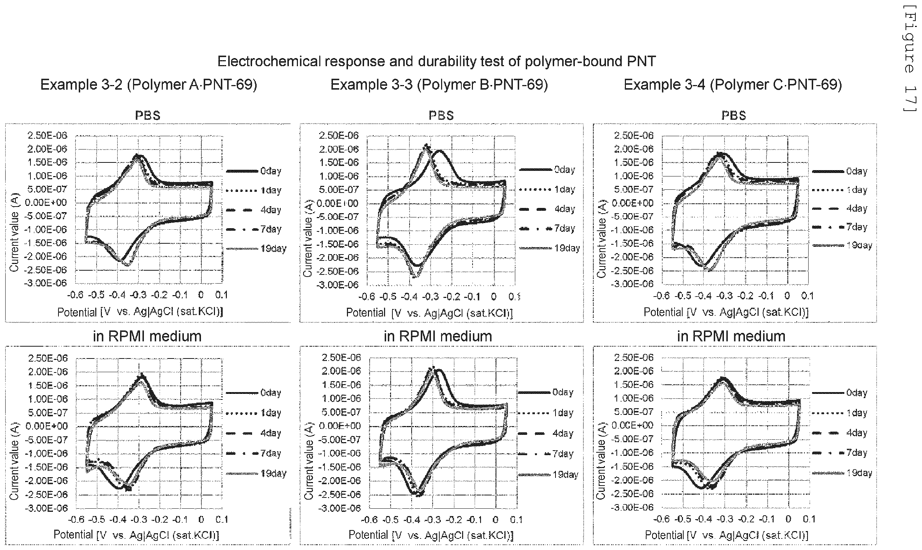

The results are shown in Figure 17. No large decrease in redox peak was seen both in PBS and in RPMI medium on day 19 after the start of preservation at 37°C. This demonstrates that the polymer-bound PNT was neither markedly deactivated nor leaked from the electrode.

### <Sensor evaluation test>

### (1) Preparation of reagent solution

Reagents shown below were mixed at the following final concentrations and reacted for approximately 1 hour to prepare a reagent solution. The carbon dispersion was used after being treated in an ultrasonic bath for approximately 10 minutes before use.
Sodium phosphate buffer solution (pH 6.5), final concentration: 5 mM
Carbon dispersion, final concentration: 5 mg/mL
Each polymer-bound PNT, final concentration: 2 (absorbance at 608 nm)
FAD-dependent glucose dehydrogenase (GDH GLD1: manufactured by BBI International), final concentration: 2000 U/mL
25% glutaraldehyde solution (manufactured by FUJIFILM Wako Pure Chemical Corp.), final concentration: 0.01% (wt/v)

The sodium phosphate buffer solution was prepared with disodium hydrogen phosphate (manufactured by FUJIFILM Wako Pure Chemical Corp.) and sodium dihydrogen phosphate (manufactured by FUJIFILM Wako Pure Chemical Corp.). The carbon dispersion was obtained by mixing Ketjen Black (EC300J: manufactured by Lion Specialty Chemicals Co., Ltd.) at a carbon concentration of 16 mg/mL with a 5 mg/mL hydroxypropylcellulose (NISSO HPCL manufactured by Nippon Soda Co., Ltd.) solution, and treating the mixture in an ultrasonic homogenizer for 3 minutes or longer. The concentration of each polymer-bound PNT was adjusted by diluting each solution of the polymer-bound PNT 25-fold, adding the dilution at 100 µL to a microplate, measuring an absorption spectrum using a plate reader to confirm the concentration of each PNT in the solution, and adjusting the concentration on the basis of the obtained value.

### (2) Preparation of polymer solution for protective film

A sensor was prepared in the same manner as in the "electrochemical response and durability test" mentioned above.

### (3) Preparation of electrode for sensor

A gold electrode was formed on an insulating substrate by sputtering. The reagent solution containing each polymer-bound PNT was applied at 0.4 µL to separate gold electrodes and dried for approximately 15 minutes. This operation was repeated a total of three times and dried overnight. A reagent layer was thereby formed on each gold electrode. Each gold electrode provided with the reagent layer was dipped in the polymer solution for a protective film, pulled up, and dried. This operation was repeated a plurality of times to form a protective film on each gold electrode. This process produced an electrode for a sensor comprising the reagent layer containing each polymer-bound PNT, and having the protective film with which the reagent layer was coated.

### (4) Sensor evaluation

The electrode for a sensor prepared in (3) as a working electrode, a gold electrode as a counter electrode, and a Ag/AgCl electrode (saturated KCl) (manufactured by BAS Inc.) as a reference electrode were combined to prepare an electrode portion of a three-electrode system. Time-dependent change in current was measured in PBS or RPMI medium at 37°C by amperometry using a potentiostat (manufactured by BAS Inc.). Specifically, glucose was added at a theoretical value of 50 mg/dL, 150 mg/dL, 300 mg/dL, or 500 mg/dL every 500 seconds from 1000 seconds after the start of measurement, and current response values were continuously measured. The electrode after measurement was preserved in PBS or RPMI medium of 37°C. The same measurement as above was performed on days 2 and 3 after the start of preservation. The current value at the glucose concentration of 50 mg/dL, 150 mg/dL, or 300 mg/dL was an average value calculated from five values measured on 5 seconds, 10 seconds, 15 seconds, 20 seconds, and 25 seconds immediately before the subsequent addition of glucose from after addition of glucose to be measured. The current value at the final concentration was an average value calculated from five values measured on 480 seconds, 485 seconds, 490 seconds, 495 seconds, and 500 seconds immediately before the subsequent addition of glucose from after addition of a glucose solution at a glucose concentration of 500 mg/dL. The current value at each glucose concentration was a current value subjected to background correction treatment of subtracting the current value at a glucose concentration of 0 mg/dL.

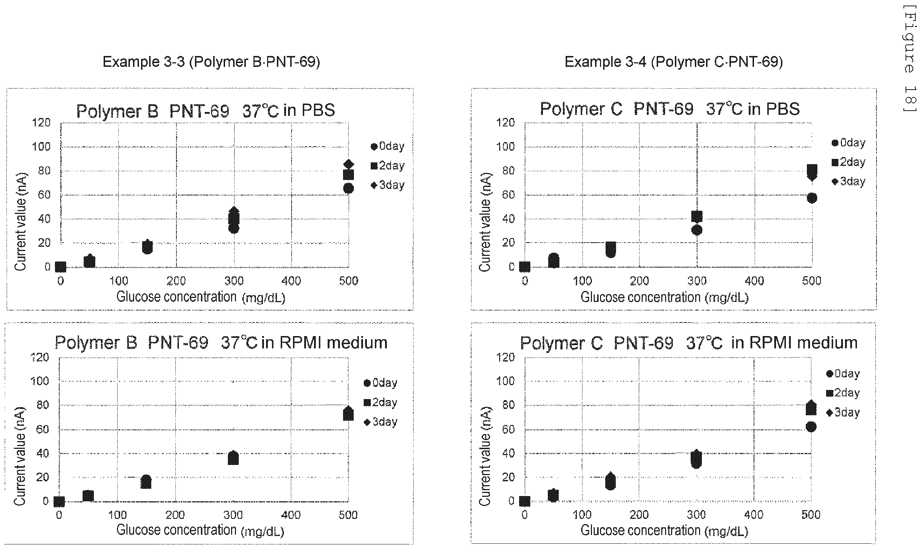

The results are shown in Figure 18. A glucose concentration-dependent elevation in current response value was observed both in PBS and in RPMI medium. This demonstrates that polymer-bound PNT has reactivity with an enzyme and functions as a glucose sensor. No marked decrease in current value was observed even on 3 days of preservation. It can thus be understood that the reactivity of polymer-bound PNT with an enzyme is maintained.

## Claims

1. A phenothiazine-based compound having a hydrophilic moiety introduced therein, the compound being represented by the general formula (2A) or (2B): 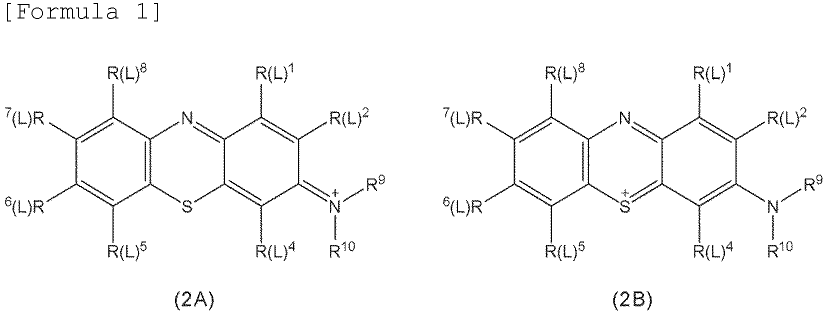 wherein
R⁹ and R¹⁰ are both substituents, or one of these moieties is a substituent, and the other moiety is a hydrogen atom,
R(L)¹, R(L)², and R(L)⁴ to R(L)⁸ are each independently [A] a hydroxy group optionally having a substituent, a carboxy group optionally having a substituent, an amino group optionally having a substituent, a linear or branched saturated or unsaturated hydrocarbon group having 1 to 6 carbon atoms and optionally having a substituent, an acyl group optionally having a substituent, or a phenyl group optionally having a substituent, [B] a group formed by the binding of a compound for hydrophilic moiety introduction to the binding substituent selected from [A], or [C] a hydrogen atom or a halogen atom,
on the proviso that at least one of R(L)¹, R(L)², and R(L)⁴ to R(L)⁸ is the group [B].

2. The phenothiazine-based compound having a hydrophilic moiety introduced therein according to claim 1, wherein the substituent represented by each of R⁹ and R¹⁰ is an alkyl group having 1 to 6 carbon atoms.

3. The phenothiazine-based compound having a hydrophilic moiety introduced therein according to claim 1, wherein at least one of R(L)¹, R(L)², and R(L)⁴ to R(L)⁸ is a group formed by the binding of a compound for hydrophilic moiety introduction to a binding substituent having at least one reactive group selected from the group consisting of a carboxy group or an active ester form thereof, an amino group, a thiol group, a formyl group, an epoxy group, and a maleimide group.

4. The phenothiazine-based compound having a hydrophilic moiety introduced therein according to claim 1, wherein at least one of R(L)¹, R(L)², and R(L)⁴ to R(L)⁸ is a group formed by the binding of a compound for hydrophilic moiety introduction to a binding substituent which is an amino group having a substituent, the binding substituent being represented by -NR¹¹R¹² (wherein one of R¹¹ and R¹² is a reactive N substituent, and the other moiety is a hydrogen atom or a nonreactive N substituent).

5. The phenothiazine-based compound having a hydrophilic moiety introduced therein according to claim 1, wherein at least R(L)⁶ is a group formed by the binding of a compound for hydrophilic moiety introduction to the binding substituent.

6. The phenothiazine-based compound having a hydrophilic moiety introduced therein according to claim 1, wherein the compound is represented by the general formula (2A-2) or (2B-2): 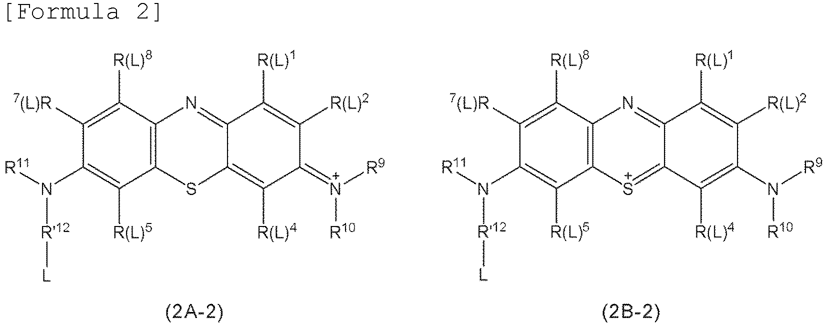 wherein R¹¹ is a hydrogen atom or a nonreactive N substituent, R'¹² is a group derived from a reactive N substituent, L is the hydrophilic moiety derived from the compound for hydrophilic moiety introduction, and the other symbols are as defined in the general formulas (2A) and (2B).

7. The phenothiazine-based compound having a hydrophilic moiety introduced therein according to claim 1, wherein the hydrophilic moiety derived from the compound for hydrophilic moiety introduction comprises at least one member selected from the group consisting of a hydroxy group, an amino group, a carboxy group, a sulfo group, a quaternary ammonium cation, and groups derived therefrom.

8. The phenothiazine-based compound having a hydrophilic moiety introduced therein according to claim 1, wherein the hydrophilic moiety derived from the compound for hydrophilic moiety introduction comprises an oxyethylene chain.

9. The phenothiazine-based compound having a hydrophilic moiety introduced therein according to claim 1, wherein the hydrophilic moiety derived from the compound for hydrophilic moiety introduction has at least one reactive group selected from the group consisting of a carboxy group or an active ester form thereof, an amino group, a thiol group, a formyl group, an epoxy group, and a maleimide group.

10. A phenothiazine-based compound bound to a polymer, the compound being represented by the general formula (3A) or (3B): 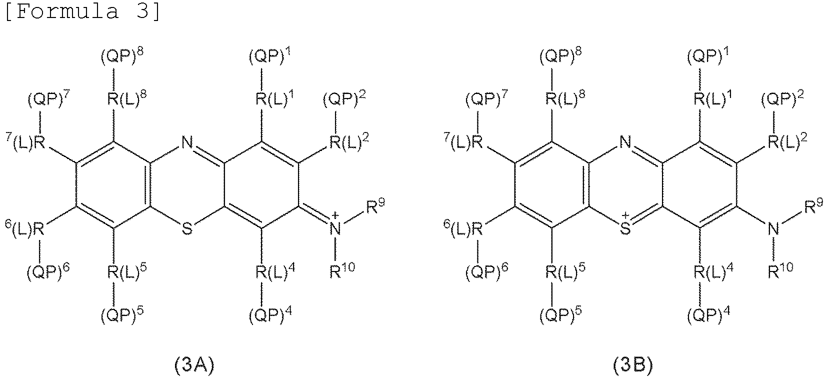 wherein
R⁹ and R¹⁰ are both substituents, or one of these moieties is a substituent, and the other moiety is a hydrogen atom,
R(L)¹, R(L)², and R(L)⁴ to R(L)⁸ are each independently [A] a hydroxy group optionally having a substituent, a carboxy group optionally having a substituent, an amino group optionally having a substituent, a linear or branched saturated or unsaturated hydrocarbon group having 1 to 6 carbon atoms and optionally having a substituent, an acyl group optionally having a substituent, or a phenyl group optionally having a substituent, [B] a group formed by the binding of a compound for hydrophilic moiety introduction to the binding substituent selected from [A], [C] a hydrogen atom or a halogen atom, or [D] a group derived from either the binding substituent selected from [A] or the group [B],
(QP)¹, (QP) ², and (QP)⁴ to (QP)⁸ are each independently a polymeric structure derived from a high molecular weight polymer which is present when their corresponding R(L)¹, R(L)², and R(L)⁴ to R(L)⁸ are the group [D], and
on the proviso that at least one of (QP)¹, (QP)², and (QP)⁴ to (QP)⁸ is present.

11. The phenothiazine-based compound bound to a polymer according to claim 10, wherein the substituent represented by each of R⁹ and R¹⁰ is an alkyl group having 1 to 6 carbon atoms.

12. The phenothiazine-based compound bound to a polymer according to claim 10, wherein at least one of R(L)¹, R(L)², and R(L)⁴ to R(L)⁸ is a group derived from either a binding substituent having at least one reactive group selected from the group consisting of a carboxy group or an active ester form thereof, an amino group, a thiol group, a formyl group, an epoxy group, and a maleimide group, or a group formed by the binding of a compound for hydrophilic moiety introduction to the binding substituent.

13. The phenothiazine-based compound bound to a polymer according to claim 10, wherein at least one of R(L)¹, R(L)², and R(L)⁴ to R(L)⁸ is a group derived from either a binding substituent which is an amino group having a substituent, the binding substituent being represented by -NR¹¹R¹² (wherein one of R¹¹ and R¹² is a reactive N substituent, and the other moiety is a hydrogen atom or a nonreactive N substituent), or a group formed by the binding of a compound for hydrophilic moiety introduction to the binding substituent.

14. The phenothiazine-based compound bound to a polymer according to claim 10, wherein at least R(L)⁶ is a group derived from either a binding substituent or a group formed by the binding of a compound for hydrophilic moiety introduction to the binding substituent.

15. The phenothiazine-based compound bound to a polymer according to claim 10, wherein at least R(L)⁶ is a group derived from a group formed by the binding of a compound for hydrophilic moiety introduction to the binding substituent.

16. The phenothiazine-based compound bound to a polymer according to claim 10, wherein the compound is represented by the general formula (3A-2) or (3B-2): 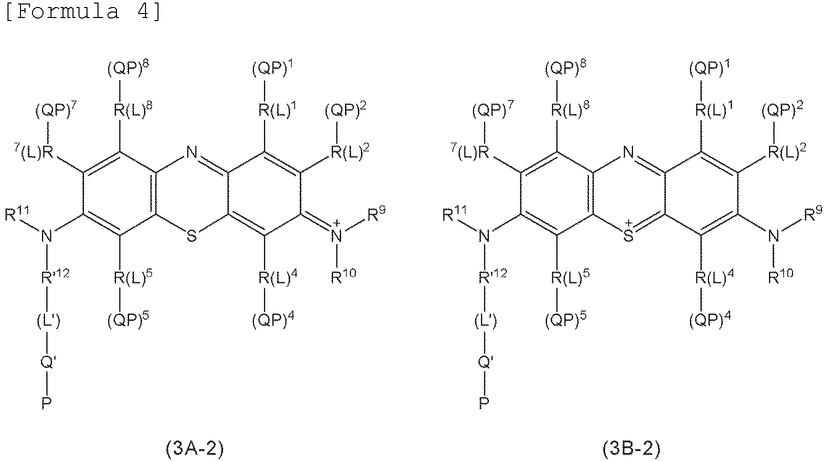 wherein R¹¹ is a hydrogen atom or a nonreactive N substituent, R'¹² is a group derived from a reactive N substituent, (L') is optionally present and is a group derived from a compound for hydrophilic moiety introduction, Q' is a group derived from a reactive group of a high molecular weight polymer, P is a backbone of the high molecular weight polymer, and the other symbols are as defined in the general formulas (3A) and (3B).

17. The phenothiazine-based compound bound to a polymer according to claim 10, wherein the hydrophilic moiety derived from the compound for hydrophilic moiety introduction comprises at least one member selected from the group consisting of a hydroxy group, an amino group, a carboxy group, a sulfo group, a quaternary ammonium cation, and groups derived therefrom.

18. The phenothiazine-based compound bound to a polymer according to claim 10, wherein the hydrophilic moiety derived from the compound for hydrophilic moiety introduction comprises an oxyethylene chain.

19. The phenothiazine-based compound bound to a polymer according to claim 10, wherein at least one of R(L)¹, R(L)², and R(L)⁴ to R(L)⁸ is a group derived from a group formed by the binding of a compound for hydrophilic moiety introduction having at least one reactive group selected from the group consisting of a carboxy group or an active ester form thereof, an amino group, a thiol group, a formyl group, an epoxy group, and a maleimide group to the binding substituent.

20. The phenothiazine-based compound bound to a polymer according to claim 10, wherein the high molecular weight polymer is at least one member selected from the group consisting of an amino acid-based polymer, an imine-based polymer, an ethylene-based polymer, and a protein.

21. The phenothiazine-based compound bound to a polymer according to claim 10, wherein the high molecular weight polymer is a cationic high molecular weight polymer.

22. A reagent layer comprising a phenothiazine-based compound having a hydrophilic moiety introduced therein according to any one of claims 1 to 9, or a phenothiazine-based compound bound to a polymer according to any one of claims 10 to 21.

23. The reagent layer according to claim 22, further comprising an oxidoreductase that oxidizes or dehydrogenates an analyte.

24. The reagent layer according to claim 22, wherein the oxidoreductase is cross-linked to the phenothiazine-based compound bound to a polymer.

25. An electrochemical sensor for detecting or quantifying an analyte, the electrochemical sensor having a working electrode, a counter electrode, and the reagent layer according to claim 22 disposed on the working electrode.

26. The electrochemical sensor according to claim 25, further having a reference electrode.

27. The electrochemical sensor according to claim 25, further having a protective film with which at least the reagent layer is coated.
